# EUROPÄISCHE PATENTSCHRIFT

(11) **EP 2 952 516 B1**
(45) Veröffentlichungstag und Bekanntmachung des Hinweises auf die Patenterteilung: **09.08.2017**
(21) Anmeldenummer: 15170409.5
(22) Anmeldetag: 03.06.2015
(51) Int. Cl.: C07F 7/18, A61K 6/00

(54) **POLYSILOXANVERBINDUNG UND DARAUS HERSTELLBARE DENTALMATERIALIEN**
POLYSILOXANE COMPOUND AND DENTAL MATERIALS THAT CAN BE PRODUCED FROM SAME
COMPOSÉ DE POLYSILOXANE ET MATÉRIAUX DENTAIRES AINSI FABRIQUÉS

(30) Priorität: 03.06.2014 DE 102014210432
(43) Veröffentlichungstag der Anmeldung: 09.12.2015
(73) Patentinhaber: VOCO GmbH, 27472 Cuxhaven (DE)
(72) Erfinder: Lübbe, Gerrit, 27476 Cuxhaven (DE)
(74) Vertreter: Eisenführ Speiser

(56) Entgegenhaltungen:
- JP-A- H1 087 671

## Beschreibung

Die vorliegende Erfindung betrifft eine Polysiloxanverbindung umfassend spezifische Siloxaneinheiten, härtbare Dentalmaterialien umfassend eine oder mehr als eine erfindungsgemäße Polysiloxanverbindung, gehärtete Dentalmaterialien erhältlich aus den erfindungsgemäßen härtbaren Dentalmaterialien, ein Verfahren zur Herstellung erfindungsgemäßer Polysiloxanverbindungen, ein Verfahren zur Herstellung erfindungsgemäßer härtbarer Dentalmaterialien und ein Verfahren zur Herstellung erfindungsgemäßer gehärteter Dentalmaterialien. Weitere Aspekte der vorliegenden Erfindung und deren bevorzugte Ausgestaltungen ergeben sich aus der folgenden Beschreibung, den Ausführungsbeispielen und den Ansprüchen.

Polysiloxanverbindungen im Sinne des vorliegenden Textes besitzen mindestens eine oder mehrere Ketten, die alternierend angeordnete und miteinander verbundene Siliziumatome und Sauerstoffatome besitzen, wobei an den Siliziumatomen organische Gruppen (organische Seitenketten) gebunden sind. Diese organischen Gruppen können sehr unterschiedlich sein und dadurch zu einer Vielzahl von Polysiloxanverbindungen mit unterschiedlichen Eigenschaften führen. Häufig besitzen diese organischen Gruppen eine oder mehrere organisch polymerisierbare Gruppen (d.h. reaktive Gruppen), die sich mit z.B. einer oder mehreren organisch polymerisierbaren Gruppen einer anderen Polysiloxanverbindung umsetzen können und dadurch vernetzte/polymerisierte Polysiloxanverbindungen bilden.

Polysiloxanverbindungen sind seit langem bekannt und z.B. erhältlich durch Hydrolyse und Kondensation von Silanen mit hydrolysierbaren Gruppen (siehe z.B. DE 27 58 414 A1) oder durch Hydrosilylierung von Allyl- oder Vinylverbindungen mit SiH-haltigen Verbindungen. Polysiloxanverbindungen können zu einer Vielzahl von Produkten weiterverarbeitet werden, wie z.B. Überzüge, Beschichtungen, Membranen oder Bulkmaterialien. Diese Weiterverarbeitung beruht dabei häufig auf einer Vernetzungsreaktion organisch polymerisierbarer Gruppen in den Polysiloxanverbindungen (z.B. (Meth)acrylatgruppen) und der daraus resultierenden Bildung vernetzter Polysiloxanverbindungen.

Eine spezifische Gruppe von Polysiloxanverbindungen enthält in den organischen Gruppen (Seitenketten) neben einer organisch polymerisierbaren Gruppe zusätzliche freie polare funktionelle Gruppen wie z.B. Hydroxy- oder Carboxygruppen.

So betrifft DE 44 16 857 C1 hydrolysierbare und polymerisierbare Silane, Verfahren zu deren Herstellung sowie deren Verwendung zur Herstellung von Kieselsäure(hetero)polykondensaten und (Hetero)Polymerisaten. Hydrolysierbare, organisch modifizierte Silane finden eine breite Anwendung bei der Herstellung kratzfester Beschichtungen für die unterschiedlichsten Substrate, für die Herstellung von Füllstoffen, von Klebe- und Dichtungsmassen oder von Formkörpern.

Unter Berücksichtigung der Lehre in DE 44 16 857 C1 wurde bei der Vorbereitung eigener Untersuchungen der Gedankenansatz berücksichtigt, dass die in DE 44 16 857 C1 offenbarten Kieselsäure(hetero)polykondensaten (Polysiloxanverbindungen) außerdem in härtbaren Dentalmaterialien eingesetzt werden können. Es wurde berücksichtigt, dass diese Polysiloxanverbindungen freie polare funktionelle Gruppen (z.B. Carboxy- oder Hydroxygruppen) umfassen, die in der Lage sind, geeignete Metallionen / Übergangsmetallionen zu komplexieren (z.B. Ionen des Titans, Zirkoniums oder Zinns). In härtbaren Dentalmaterialien kann sich dies positiv auf die Röntgenopazität, die Kontakttoxizität und auf den Brechungsindex eines entsprechenden härtbaren bzw. gehärteten Dentalmaterials auswirken.

DE 198 60 364 C2 betrifft polymerisierbare Dentalmassen auf der Basis von zur Aushärtung befähigten Siloxanverbindungen, deren Verwendung und Herstellung. Bei der Vorbereitung eigener Untersuchungen wurde berücksichtigt, dass in DE 198 60 364 C2 die Herstellung von cyclischen Polysiloxanen und deren Verwendung als Basis für polymerisisierbare Dentalmassen beschrieben wird, Sie sollen trotz hoher Dichte an zur Polymerisation befähigten Gruppen eine niedrige Viskosität aufweisen, die eine hohe Füllstoffaufnahme ermöglicht welche zu Massen mit geringen Polymerisationsschrumpf führen. Auch hier befinden sich in den organischen Seitenketten der beschriebenen Polysiloxane neben den polymerisierbaren Einheiten, freie polare Funktionen.

Die freien polaren funktionellen Gruppen, z.B. in den vorstehend genannten Polysiloxanverbindungen führen jedoch regelmäßig auch zu unerwünschten Eigenschaften. So haben jetzt eigene Untersuchungen ergeben, dass die durch die (freien) polaren funktionellen Gruppen hervorgerufene Hydrophilie der Polysiloxanverbindungen zu einer erhöhten Wasseraufnahme in Gegenwart von Feuchtigkeit führt, wodurch die Nassfestigkeit des härtbaren Dentalmaterials in nachteiliger Weise verringert wird. Vermutlich durch Aufbau interner Wasserstoffbrückenbindungen kommt es zu einer Erhöhung der Viskosität. Dies wirkt sich dann negativ auf die Handhabbarkeit bei der Herstellung der härtbaren Dentalmassen aus.

Es besteht ein beträchtliches Bedürfnis seitens Dentalmedizinern und der Dentalindustrie, Polysiloxanverbindungen weiter an die Anforderungen an ein modernes (härtbares bzw. gehärtetes) Dentalmaterial anzupassen und die vorstehend genannten Nachteile zu minimieren. Derart angepasste Polysiloxanverbindungen sollen dabei dentalmedizinisch verbesserte physikalische Eigenschaften besitzen (bzw. zu dentalmedizinisch verbesserten physikalischen Eigenschaften entsprechender härtbarer/gehärteter Dentalmaterialien führen), z.B. einen geringeren Polymerisationsschrumpf beim Polymerisieren/Vernetzen der Polysiloxanverbindungen (d.h. beim Aushärten), eine erhöhte Festigkeit und/oder eine begrenzte Wasseraufnahme bei gleichzeitig komfortabler Konsistenz des härtbaren Dentalmaterials.

Erste Erfolge bei der Verbesserung der Polysiloxane konnten durch Addition bzw. Substitution unterschiedlicher Substrate an die freien polaren Funktionalitäten der oben beschrieben speziellen Polysiloxane erreicht werden.

EP 1 874 847 B1 betrifft ein Verfahren zur Herstellung von Silanen mit zwei, drei oder sogar mehr Struktureinheiten, die über eine urethan-, säureamid- und/oder carbonsäureestergruppenhaltige Brücke miteinander verknüpft sind und von denen jede mindestens einen organisch polymerisierbaren Rest und mindestens einen Silylrest enthält. Diese Silane sollen sich insbesondere zur Modifizierung der Eigenschaften von Kieselsäure(hetero)polykondensaten und silylgruppenhaltigen, organischen Polymeren (ORMOCER®en) eignen. Das offenbarte Verfahren soll sich auch zur Verbrückung bereits vorkondensierter Kieselsäure(hetero)polykondensate eignen.

Unter Berücksichtigung der Lehre in EP 1 874 847 B1 wurde bei der Vorbereitung eigener Untersuchungen der Gedankenansatz berücksichtigt, dass die in EP 1 874 847 B1 offenbarten Kieselsäure(hetero)polykondensate (Polysiloxanverbindungen) eine freie Hydroxygruppe (d.h. eine freie polare funktionelle Gruppe) besitzen. Diese freien Hydroxygruppen können mit einem Dicarbonsäurederivat oder Diisocyanat so reagieren, dass Hydroxylgruppen mit einem Dicarbonsäurederivat bzw. Diisocyanat eine Verknüpfung (Verbrückung) bilden (vgl. das nachfolgende exemplarische Schema (a)). Solche verknüpften Polysiloxanverbindungen (Verbindung (101) im exemplarischen Schema (a)) besitzen ein deutlich höheres Molekulargewicht, ohne dabei die Doppelbindungsdichte (bedingt durch die organisch polymerisierbaren (Meth)acrylatgruppen) wesentlich zu verringern. Unter Doppelbindungsdichte versteht der Fachmann den Quotienten aus der Anzahl der polymerisierbaren Doppelbindungen in einer Verbindung und dem Molekulargewicht dieser Verbindung. Das höhere Molekulargewicht wirkt sich positiv auf die Biokompatibilität und den Polymerisationsschrumpf beim Vernetzen der verknüpften Polysiloxanverbindungen aus. Gleichzeitig konnte die Hydrophobizität der Polysiloxanverbindungen gesteigert werden. Es hat sich in eigenen Untersuchungen jedoch gezeigt, dass sich das höhere Molekulargewicht nachteilig auf die Viskosität der verknüpften Polysiloxanverbindungen (und damit auf die Verarbeitbarkeit bei der Herstellung des härtbaren Dentalmaterials) auswirkt. Die Viskosität steigt mit dem Grad der Verknüpfung, d.h. mit dem Molekulargewicht deutlich an, so dass eine tolerierbare Verarbeitbarkeit bei der Herstellung eines entsprechenden härtbaren Dentalmaterials, das solche verknüpften Polysiloxanverbindungen umfasst, schon bei recht geringem Verknüpfungsgrad nicht mehr zufriedenstellend gegeben ist.

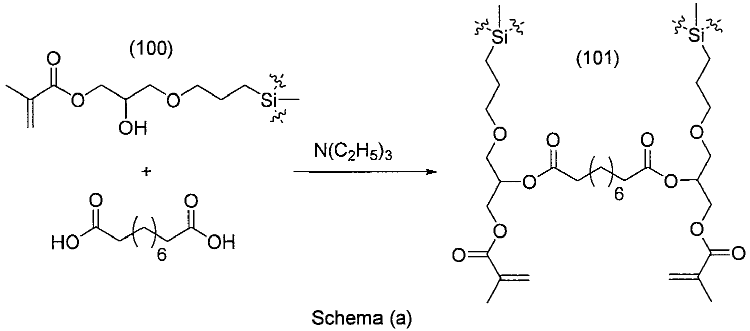

EP 1 685 182 B1 betrifft Silane und daraus gebildete Kieselsäurepolykondensate und -teilkondensate, in denen ein an einem Silicium gebundener, organischer Rest vorhanden ist, der verzweigt ist und and jedem der beiden Zweige mindestens eine eigenständig organisch polymerisierbare Gruppe trägt oder an einem der beiden Zweige eine solche Gruppe, am anderen einen Rest mit einem weiteren Siliziumatom aufweist.

Auch die in EP 1 685 182 B1 offenbarten Polysiloxanverbindungen umfassen freie polare funktionelle Gruppen in Form von Hydroxygruppen. Unter Berücksichtigung der Lehre in EP 1 685 182 B1 wurde bei der Vorbereitung eigener Untersuchungen der Gedankenansatz berücksichtigt, dass durch Reaktion Carbonsäure- oder Isocyanatderivate, die ihrerseits ebenfalls polymerisierbare Doppelbindungen in Form organisch polymerisierbare Gruppen umfassen (z.B. (Meth)acrylatgruppen), an freie polare funktionelle Gruppen geknüpft werden können (vgl. das nachfolgende exemplarische Schema (b)). Die Reaktionsprodukte (vgl. Verbindung (102) im exemplarischen Schema (b)) besitzen regelmäßig eine erhöhte Festigkeit bei gleichzeitig gesteigerter Hydrophobizität und verbesserter Biokompatibilität durch das erhöhte Molekulargewicht.

Die eigenen Untersuchungen haben jedoch auch gezeigt, dass das Einführen zusätzlicher polymerisierbarer Doppelbindungen in Form organisch polymerisierbarer Gruppen zu einem erhöhten Polymerisationsschrumpf beim Vernetzen der Polysiloxanverbindungen führt, da sich die Doppelbindungsdichte deutlich erhöht, die Erhöhung des Molekulargewichtes aber nur vergleichsweise gering ist.

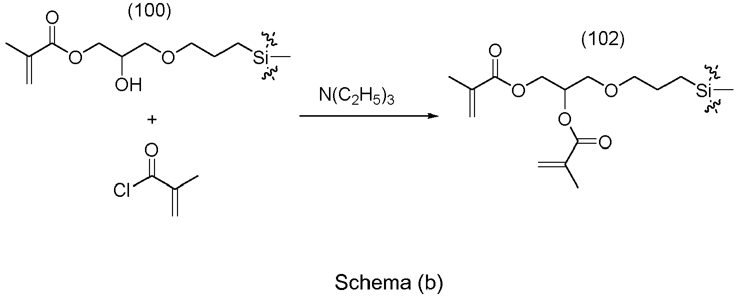

WO 2013/041723 A1 offenbart hydrolysierbare und polymerisierbare Silane (einschließlich Kieselsäurepolykondensate, d.h. Siloxane) mit einstellbarer räumlicher Verteilung der funktionellen Gruppen sowie deren Verwendung. Die in WO 2013/041723 A1 offenbarte Lehre betrifft ein Verfahren zur Kettenverlängerung von über Kohlenstoff an Silizium gebundenen Resten in Silanen oder Siloxanen.

WO 2013/053693 A1 offenbart Kieselsäurepolykondensate (Siloxane) mit cyclischen olefinhaltigen Strukturen und Verfahren zu deren Herstellung sowie deren Verwendung. WO 2013/053693 A1 offenbart, dass sich Polymerwerkstoffe mit in weiten Grenzen einstellbaren E-Moduli bei hoher elastischer Dehnung (d.h. ohne Sprödverhalten) und damit einer hohen Bruchzähigkeit aus Kieselsäure(hetero)polykondensaten mit cyclischen olefinhaltigen Strukturen herstellen lassen.

JP H1087671A (Daikin Ind Ltd) offenbart Silan-basierte Verbindungen, die gemäß dem veröffentlichten Abstract dazu geeignet sein sollen, die Wasserbeständigkeit eines dentalen Kompositharzes und die Haftfestigkeit des Harzes zu verbessern.

Es war die Aufgabe der vorliegenden Erfindung, eine Polysiloxanverbindung bereitzustellen, die die vorstehend genannten Nachteile nicht oder zumindest nur noch abgeschwächt aufweist. Vorzugsweise sollte die bereitzustellende Polysiloxanverbindung zu einer, mehreren oder besonders bevorzugt zu sämtlichen der nachstehend genannten Eigenschaften führen, insbesondere bei Einsatz in einem härtbaren bzw. gehärteten Dentalmaterial (und insbesondere im Vergleich mit Polysiloxanverbindungen nach Art der in den Schemata (a) und (b) als Edukt gezeigten und eine freie polare funktionelle Gruppe tragenden Polysiloxanverbindung):
- eine gute Viskosität der Polysiloxanverbindungen (die Viskosität sollte 50 Pa*s oder weniger betragen bei einer Temperatur von 25°C) und eine damit einhergehende hervorragende Verarbeitbarkeit bei der Herstellung eines die Polysiloxanverbindungen enthaltenden härtbaren Dentalmaterials,
- eine gute Hydrophobizität,
- eine gute Festigkeit, insbesondere eine gute Biegefestigkeit,
- einen sehr geringen Polymerisationsschrumpf beim Vernetzen der Polysiloxanverbindungen, d.h. beim Aushärten des härtbaren Dentalmaterials,
- eine gute Biokompatibilität,
- einen Brechungsindex besitzen, der nahezu identisch ist mit dem Brechungsindex üblicher dentaler Gläser (vorzugsweise sogar nahezu identisch ist mit dem Brechungsindex besonders röntgenopaker Barium- bzw. Zirkoniumgläser)

Die vorstehend genannte Aufgabe wird erfindungsgemäß gelöst durch eine Polysiloxanverbindung umfassend
(A) ein, zwei, drei oder mehr als drei strukturell jeweils identische erste Siloxaneinheiten ausgewählt aus der Gruppe bestehend aus Siloxaneinheiten der allgemeinen Formel (I) 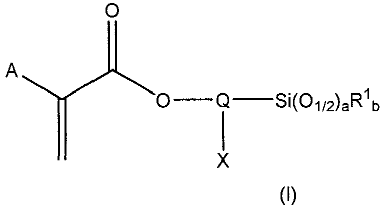
   wobei für die Struktureinheiten A, Q, X und R¹ in jeder der strukturell identischen ersten Siloxaneinheiten unabhängig voneinander gilt:
   A bedeutet H oder CH₃,
   Q bedeutet eine den Substituenten X tragende Verknüpfungsgruppe,
   R¹ bedeutet einen Alkylrest mit insgesamt 1 bis 4 Kohlenstoffatomen und
   X ist ausgewählt aus der Gruppe bestehend aus
      verzweigter, gesättigter, unsubstituierter Alkylrest mit insgesamt 7 bis 18 Kohlenstoffatomen,
      unverzweigter, gesättigter, unsubstituierter Alkylrest mit insgesamt 7 bis 18 Kohlenstoffatomen,
      unsubstituierter oder alkylsubstituierter Arylrest mit insgesamt 10 bis 18 Kohlenstoffatomen, wobei der Alkylsubstituent im alkylsubstituierten Arylrest
         verzweigt und gesättigt
            oder
         unverzweigt und gesättigt ist
         und
      Z-(CO)-R², wobei hierin für die Struktureinheiten Z und R² unabhängig voneinander und unabhängig von der Bedeutung der Struktureinheiten A, Q und R¹ gilt:
         Z bedeutet O, S oder NH, vorzugsweise O
            und
         R² ist ausgewählt aus der Gruppe bestehend aus
            verzweigter, gesättigter, unsubstituierter Alkylrest mit insgesamt 6 bis 18 Kohlenstoffatomen,
            unverzweigter, gesättigter, unsubstituierter Alkylrest mit insgesamt 6 bis 18 Kohlenstoffatomen,
            unsubstituierter oder alkylsubstituierter Arylrest mit insgesamt 9 bis 18 Kohlenstoffatomen, wobei der Alkylsubstituent im alkylsubstituierten Arylrest
               verzweigt und gesättigt
                  oder
               unverzweigt und gesättigt ist,
            wobei
               - b: 0, 1 oder 2 bedeutet und
               - a: 3-b bedeutet.

Die erfindungsgemäße Polysiloxanverbindung ist ein Dimer, Oligomer oder Polymer, das eine erste Siloxaneinheit der allgemeinen Formel (I) umfasst oder zwei, drei oder mehr als drei strukturell jeweils identische erste Siloxaneinheiten der allgemeinen Formel (I) umfasst. In vorstehender Strukturformel (sowie in allen entsprechenden nachfolgenden Strukturformeln) bedeutet O_{1/2} die Sauerstoffverknüpfung zum nächsten Siliziumatom in einer erfindungsgemäßen Polysiloxanverbindung.

R¹ bedeutet vorzugsweise Methyl, Ethyl, n-Propyl, iso-Propyl, n-Butyl, iso-Butyl, sec-Butyl oder tert-Butyl.

In der erfindungsgemäßen Polysiloxanverbindung kann ein "Alkylrest" (bei ausreichender Zahl von Kohlenstoffatomen) ein Cycloalkylrest sein oder einen Cycloalkylrest umfassen. D.h., ein "Alkylrest" einer erfindungsgemäßen Polysiloxanverbindung
- umfasst oder umfasst nicht einen Cycloalkylrest
   oder
- ist oder ist nicht ein Cycloalkylrest.

Ein Kohlenstoffatom des Alkylrests (wie oben definiert) kann mit anderen Worten mit einem anderen Kohlenstoffatom dieses Alkylrests unter Bildung eines Ringes verknüpft sein.

Die eine erste Siloxaneinheit bzw. die mehreren strukturell jeweils identischen ersten Siloxaneinheiten der allgemeinen Formel (I) besitzen jeweils eine organisch polymerisierbare Gruppe in Form einer (Meth)acrylatgruppe (A bedeutet H oder CH₃). Die Doppelbindungen dieser (Meth)acrylatgruppen erlauben eine Vernetzung/Polymerisation mit entsprechenden weiteren organisch polymerisierbaren Gruppen (bzw. deren Doppelbindungen). Vorzugsweise ist die Doppelbindungsdichte in der erfindungsgemäßen Polysiloxanverbindung ausschließlich durch die (Meth)acrylatgruppen in den jeweiligen Siloxaneinheiten der allgemeinen Formel (I) bestimmt. Das bedeutet, dass der Substituent X (wie vorstehend definiert) vorzugsweise keine zusätzlichen organisch polymerisierbaren Doppelbindungen (in Form organisch polymerisierbarer Gruppen) enthält (wie sie z.B. in zusätzlichen (Meth)acrylatgruppen enthalten wären). Die Substituenten X sind vielmehr vorzugsweise so ausgewählt, dass sie keine organisch polymerisierbaren Doppelbindungen enthalten, aber das Molekulargewicht der erfindungsgemäßen Polysiloxanverbindung deutlich erhöhen bei gleichzeitiger Steigerung der Hydrophobizität. Angestrebte Eigenschaften werden so (besser) erreicht.

Die Doppelbindungsdichte in den erfindungsgemäßen Polysiloxanverbindungen ist wegen der Anwesenheit der Substituenten X vergleichsweise reduziert (z.B. verglichen mit einer nicht erfindungsgemäßen Polysiloxanverbindung, deren Siloxaneinheiten anstelle des Substituenten X jeweils eine Hydroxygruppe umfassen, vgl. lediglich beispielhaft die nicht erfindungsgemäße Verbindung (100) im exemplarischen Schema (a)). Wegen der Anwesenheit der Substituenten X besitzt die erfindungsgemäße Polysiloxanverbindung ein vergleichsweise höheres Molekulargewicht.

Es war daher zu erwarten, dass die vergleichsweise reduzierte Doppelbindungsdichte zu einer vergleichsweise schlechteren Vernetzung/Polymerisation führt (z.B. verglichen mit einer nicht erfindungsgemäßen Polysiloxanverbindung, deren Siloxaneinheiten anstelle des Substituenten X jeweils eine Hydroxygruppe umfassen, vgl. lediglich beispielhaft Verbindung (100) im exemplarischen Schema (a)) und damit zu einer vergleichsweise niedrigeren Festigkeit eines entsprechenden gehärteten Dentalmaterials. Es war zudem zu erwarten, dass das vergleichsweise höhere Molekulargewicht durch die Anwesenheit des Substituenten X nachteilige Auswirkungen auf die Materialeigenschaften wie beispielsweise die Viskosität der erfindungsgemäßen Polysiloxanverbindung hat (z.B. eine vergleichsweise Erhöhung der Viskosität) (erneut z.B. verglichen mit einer nicht erfindungsgemäßen Polysiloxanverbindung, deren Siloxaneinheiten anstelle des Substituenten X jeweils eine Hydroxygruppe umfassen, vgl. erneut lediglich beispielhaft Verbindung (100) im exemplarischen Schema (a)) und damit eine gute Verarbeitbarkeit bei der Herstellung eines entsprechenden härtbaren Dentalmaterials nicht mehr gewährleistet ist.

Entgegen diesen Erwartungen hat sich in eigenen Untersuchungen jedoch überraschend gezeigt, dass
- sich die Festigkeit, insbesondere die Biegefestigkeit des entsprechenden gehärteten Dentalmaterials (und damit die Vernetzung/Polymerisation der erfindungsgemäßen Polysiloxanverbindung) auf einem sehr guten Niveau verbleibt und in vielen Fällen sogar erhöht ist,
- die Viskosität der erfindungsgemäßen Polysiloxanverbindung im Vergleich nicht nachteilig verändert ist (nämlich allenfalls unwesentlich erhöht ist) und damit
- deren Verarbeitbarkeit bei Herstellung der entsprechenden härtbaren Dentalmaterialien noch immer gewährleistet ist.

Die überraschenden Ergebnisse lassen sich derzeit noch nicht komplett erklären. Aus den eigenen Untersuchungen resultieren jedoch eine Reihe von Vermutungen und Erklärungsansätzen:

Es ist zu vermuten, dass die oben definierten Substituenten X zu einer intramolekularen räumlichen Aufweitung innerhalb der erfindungsgemäßen Polysiloxanverbindung führen. Dies könnte wiederum zur Folge haben, dass die Zugänglichkeit/Reaktivität der organisch polymerisierbaren Doppelbindungen in den (Meth)acrylatgruppen erhöht wird, so dass eine größere Anzahl dieser (Meth)acrylatgruppen für eine Vernetzungs-/Polymerisationsreaktionen mit anderen organisch polymerisierbaren Doppelbindungen zur Verfügung steht. Dies könnte erklären, warum trotz vergleichsweise reduzierter Doppelbindungsdichte die Festigkeit, insbesondere die Biegefestigkeit des entsprechenden gehärteten Dentalmaterials auf einem sehr guten Niveau verbleibt und in vielen Fällen sogar erhöht ist.

Es ist außerdem zu vermuten, dass die Viskosität der erfindungsgemäßen Polysiloxanverbindung nur deshalb allenfalls unwesentlich erhöht ist (gegenüber einer nicht erfindungsgemäßen Polysiloxanverbindung, deren Siloxaneinheiten anstelle des Substituenten X jeweils eine Hydroxygruppe umfassen, vgl. erneut lediglich beispielhaft Verbindung (100) im exemplarischen Schema (a)), weil eine freie polare Hydroxygruppe aufgrund intermolekularer polarer Wechselwirkungen (z.B. in Form von Wasserstoffbrückenbindungen), zusätzlich zum Molekulargewicht einen besonderen Beitrag zur Viskosität dieser Polysiloxanverbindung leistet. Eine erfindungsgemäße Polysiloxanverbindung, die eine erste oder zwei, drei oder mehr als drei identische erste Siloxaneinheiten der allgemeinen Formel (I) umfasst, besitzt in diesen Siloxaneinheiten keine freie polare Hydroxygruppe, so dass der Beitrag zur Viskosität durch intermolekulare polare Wechselwirkungen vermutlich eliminiert ist. Der Beitrag zur Viskosität durch das erhöhte Molekulargewicht wird somit vermutlich durch den Wegfall intermolekularer polarer Wechselwirkungen (bzw. deren Beitrag zur Viskosität) teilweise kompensiert, so dass allenfalls ein geringer Beitrag zur Viskosität durch das erhöhte Molekulargewicht verbleibt.

Es wird zudem derzeit vermutet, dass die vorstehend genannten, in Verbindung mit X getroffenen Vermutungen zumindest teilweise auch gelten, wenn X gleich Z-(CO)-R² bedeutet (insbesondere wenn Z Sauerstoff ist). Für R² gilt das vorstehend Gesagte entsprechend.

Erfindungsgemäße Polysiloxanverbindungen besitzen einen Brechungsindex. In eigenen Untersuchungen hat sich insoweit gezeigt, dass der Brechungsindex der erfindungsgemäßen Polysiloxanverbindungen betraglich besonders hoch ist, wenn X bzw. R² ein Arylrest (wie oben definiert, vorzugsweise ein wie vor- oder nachstehend als bevorzugt bezeichneter Arylrest) ist. Solche erfindungsgemäßen Polysiloxanverbindungen sind besonders bevorzugt. Vorzugsweise besitzen diese bevorzugten erfindungsgemäßen Polysiloxanverbindungen einen Brechungsindex im Bereich von 1,48 bis 1,54. Es war insbesondere überraschend, dass (neben diesem bevorzugten Brechungsindexbereich) gleichzeitig die gute Viskosität der erfindungsgemäßen Polysiloxanverbindung erhalten bleibt und zusätzlich die Festigkeit, insbesondere die Biegefestigkeit eines entsprechenden gehärteten Dentalmaterials auf einem sehr guten Niveau verbleibt.

Die Bereitstellung geeigneter Polysiloxanverbindungen mit spezifischen Brechungsindizes ist für dentale Anwendungen generell von großer Bedeutung. Härtbare Dentalmaterialien umfassen regelmäßig eine oder mehrere vernetzbare Verbindungen sowie Füllstoffe, insbesondere Füllstoffe in Form dentaler Gläser. Solche dentalen Gläser besitzen einen Brechungsindex. Weicht der Brechungsindex der dentalen Gläser vom Brechungsindex der vernetzbaren Verbindung(en) signifikant ab, wird das aushärtbare Dentalmaterial bei Lichthärtung nur unzureichend vom Licht durchdrungen, da eine unzureichende Transluzenz vorliegt (weitere Ausführungen zur Aushärtung unten im Text). Liegen der Brechungsindex der dentalen Gläser und der Brechungsindex der vernetzbaren Verbindung(en) hingegen eng beieinander, erhöht sich die Transluzenz des aushärtbaren Dentalmaterials, sodass eine hervorragende Lichthärtung erfolgen kann. Die vorliegende Erfindung stellt erfindungsgemäße Polysiloxanverbindungen bereit, die einen für ein Dentalglas passenden Brechungsindex besitzen. Die vorliegende Erfindung ermöglicht es, dentale Gläser mit hohem Brechungsindex als Füllstoff einzusetzen, die in härtbaren Dentalmaterialien bisher nicht sinnvoll eingesetzt werden konnten; denn erst jetzt stehen erfindungsgemäße Polysiloxanverbindungen mit einem entsprechend hohen Brechungsindex zur Verfügung. Dies betrifft speziell röntgenopake Barium- und/oder Zirkoniumgläser.

Es hat sich außerdem in eigenen Untersuchungen gezeigt, dass eine erfindungsgemäße Polysiloxanverbindung, für die gilt: X bzw. R² ist ein wie oben beschriebener Arylrest (vorzugsweise ein wie vor- oder nachstehend als bevorzugt definierter Arylrest) in einem entsprechenden gehärteten Dentalmaterial (wie nachfolgend definiert) regelmäßig zu einer erhöhten Biegefestigkeit und einem erhöhten Elastizitätsmodul führt, verglichen mit einer nicht erfindungsgemäßen Polysiloxanverbindung umfassend eine freie polare funktionelle Gruppe (vgl. erneut und lediglich beispielhaft die Verbindung (100) gemäß der exemplarischen Schemata (a) bzw. (b)).

Eigene Untersuchungen haben zusätzlich gezeigt, dass die Viskosität einer erfindungsgemäßen Polysiloxanverbindung bzw. eines entsprechenden härtbaren Dentalmaterials vergleichsweise niedrig ist, wenn X bzw. R² ein oben definierter Alkyrest (vorzugsweise ein wie vor- oder nachstehend als bevorzugt bezeichneter Alkylrest) ist (verglichen mit einer erfindungsgemäßen Polysiloxanverbindung, in der X bzw. R² ein Arylrest ist). In diesem Fall (erniedrigte Viskosität) ist das Fließverhalten dieser erfindungsgemäßen Polysiloxanverbindung bzw. eines solchen härtbaren Dentalmaterials entsprechend vergleichsweise hoch.

Die eigenen Untersuchungen haben ebenfalls gezeigt, dass erfindungsgemäße Polysiloxanverbindungen im Vergleich mit nicht erfindungsgemäßen Polysiloxanverbindung, die eine freie polare funktionelle Gruppe tragen (vgl. erneut lediglich beispielhaft Verbindung (100) gemäß der exemplarischen Schemata (a) bzw. (b)) bei Verwendung in einem gehärteten Dentalmaterial regelmäßig zu einem verringerten Polymerisationsschrumpf beim Aushärten des entsprechenden härtbaren Dentalmaterials führen.

Bevorzugt ist eine erfindungsgemäße Polysiloxanverbindung (wie vorstehend beschrieben), weiter umfassend
(B) ein, zwei, drei oder mehr als drei zweite, strukturell jeweils identische Siloxaneinheiten, die von der ersten Siloxaneinheit strukturell verschieden sind.

Besonders bevorzugt ist eine erfindungsgemäße Polysiloxanverbindung (wie vorstehend definiert, vorzugsweise wie vorstehend als bevorzugt definiert), wobei gilt:
X ist ausgewählt aus der Gruppe bestehend aus
   verzweigter, gesättigter, unsubstituierter Alkylrest mit insgesamt 11 bis 18, vorzugsweise 11 bis 14 Kohlenstoffatomen,
   unverzweigter, gesättigter, unsubstituierter Alkylrest mit insgesamt 11 bis 18, vorzugsweise 11 bis 14 Kohlenstoffatomen,
   unsubstituierter oder alkylsubstituierter Arylrest mit insgesamt 11 bis 18, vorzugsweise 11 bis 14 Kohlenstoffatomen, wobei der Alkylsubstituent im alkylsubstituierten Arylrest
      verzweigt und gesättigt
         oder
      unverzweigt und gesättigt ist
      und
   Z-(CO)-R², wobei hierin für die Struktureinheiten Z und R² unabhängig voneinander und unabhängig von der Bedeutung der Struktureinheiten A, Q und R¹ gilt:
      Z bedeutet O, S oder NH, vorzugsweise O
         und
      R² ist ausgewählt aus der Gruppe bestehend aus
         verzweigter, gesättigter, unsubstituierter Alkylrest mit insgesamt 10 bis 18, vorzugsweise 10 bis 14 Kohlenstoffatomen,
         unverzweigter, gesättigter, unsubstituierter Alkylrest mit insgesamt 10 bis 18, vorzugsweise 10 bis 14 Kohlenstoffatomen,
         unsubstituierter oder alkylsubstituierter Arylrest mit insgesamt 10 bis 18, vorzugsweise 10 bis 14 Kohlenstoffatomen, wobei der Alkylsubstituent im alkylsubstituierten Arylrest
            verzweigt und gesättigt
               oder
            unverzweigt und gesättigt ist.

Eine erfindungsgemäße Polysiloxanverbindung kann eine Vielzahl chemischer Strukturen und insbesondere Konfigurationen und Konstitutionen besitzen. Bevorzugt ist eine erfindungsgemäße Polysiloxanverbindung (wie vorstehend definiert, vorzugsweise wie vorstehend als bevorzugt definiert), wobei die Polysiloxanverbindung ausgewählt ist aus der Gruppe bestehend aus: lineares Polysiloxan, verzweigtes Polysiloxan, monocyclisches Polysiloxan, polycyclisches Polysiloxan, chemisch vernetztes Polysiloxan und Mischtypen davon, wobei das polycyclische Polysiloxan eine Käfigverbindung ist oder keine Käfigverbindung ist.

Besonders bevorzugt ist eine erfindungsgemäße Polysiloxanverbindung ausgewählt aus der Gruppe bestehend aus lineares Polysiloxan und monocyclisches Polysiloxan.

Für viele Anwendungen bevorzugt ist eine erfindungsgemäße Polysiloxanverbindung (wie vorstehend definiert, vorzugsweise wie vorstehend als bevorzugt definiert), umfassend als strukturelle Einheit (B) oder als weitere strukturelle Einheit
ein, zwei, drei oder mehr als drei strukturell identische Siloxaneinheiten, die von der ersten Siloxaneinheit strukturell verschieden sind, aber gleichermaßen ausgewählt sind aus der Gruppe bestehend aus Siloxaneinheiten der allgemeinen Formel (I) wie vor- oder nachstehend definiert (vorzugsweise wie vor- oder nachstehend als bevorzugt definiert)
   oder
ein, zwei, drei oder mehr als drei strukturell identische Siloxaneinheiten, die von der ersten Siloxaneinheit strukturell verschieden sind und die nicht ausgewählt sind aus der Gruppe bestehend aus Siloxaneinheiten der allgemeinen Formel (I) wie vor- oder nachstehend definiert (vorzugsweise wie vor- oder nachstehend als bevorzugt definiert).

Bevorzugt ist eine erfindungsgemäße Polysiloxanverbindung (wie vorstehend definiert, vorzugsweise wie vorstehend als bevorzugt definiert), wobei
- die den Substituenten X tragende Verknüpfungsgruppe Q eine das an Q gebundene Sauerstoffatom mit dem an Q gebundenen Siliziumatom verknüpfende Kohlenstoffkette umfasst, wobei
   die Kohlenstoffkette ununterbrochen oder durch ein, zwei oder mehr als zwei Heteroatome unterbrochen ist, wobei
   - die ein, zwei oder mehr als zwei Heteroatome unabhängig voneinander ausgewählt sind aus der Gruppe bestehend aus O, S und N, vorzugsweise O
und/oder
- der Substituent X unmittelbar an ein Kohlenstoffatom der kürzesten Kette gebunden ist, die das an Q gebundene Sauerstoffatom mit dem an Q gebundenen Siliziumatom verknüpft.

Die vorliegende Erfindung betrifft auch eine Polysiloxanverbindung (vorzugsweise wie vorstehend definiert, besonders bevorzugt wie vorstehend als bevorzugt definiert), umfassend
(A) ein, zwei, drei oder mehr als drei strukturell jeweils identische erste Siloxaneinheiten ausgewählt aus der Gruppe bestehend aus Siloxaneinheiten der allgemeinen Formel (la) 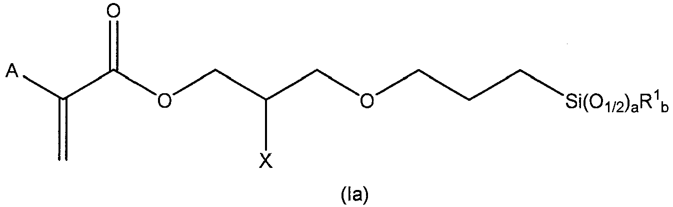 wobei für die Struktureinheiten A, X und R¹ in jeder der strukturell identischen ersten Siloxaneinheiten unabhängig voneinander gilt:
   A bedeutet H oder CH₃,
   R¹ bedeutet einen Alkylrest mit insgesamt 1 bis 4 Kohlenstoffatomen und
   X ist ausgewählt aus der Gruppe bestehend aus
      verzweigter, gesättigter, unsubstituierter Alkylrest mit insgesamt 7 bis 18 Kohlenstoffatomen, vorzugsweise 11 bis 18 Kohlenstoffatomen, besonders bevorzugt 11 bis 14 Kohlenstoffatomen,
      unverzweigter, gesättigter, unsubstituierter Alkylrest mit insgesamt 7 bis 18 Kohlenstoffatomen, vorzugsweise 11 bis 18 Kohlenstoffatomen, besonders bevorzugt 11 bis 14 Kohlenstoffatomen,
      unsubstituierter oder alkylsubstituierter Arylrest mit insgesamt 10 bis 18 Kohlenstoffatomen, vorzugsweise 11 bis 18 Kohlenstoffatomen, besonders bevorzugt 11 bis 14 Kohlenstoffatomen, wobei der Alkylsubstituent im alkylsubstituierten Arylrest
         verzweigt und gesättigt
            oder
         unverzweigt und gesättigt ist
         und
      Z-(CO)-R², wobei hierin für die Struktureinheiten Z und R² unabhängig voneinander und unabhängig von der Bedeutung der Struktureinheiten A und R¹ gilt:
         Z bedeutet O, S oder NH, vorzugsweise O
            und
         R² ist ausgewählt aus der Gruppe bestehend aus
            verzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
            unverzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
            unsubstituierter oder alkylsubstituierter Arylrest mit insgesamt 9 bis 18 Kohlenstoffatomen, vorzugsweise 10 bis 18 Kohlenstoffatomen, besonders bevorzugt 10 bis 14 Kohlenstoffatomen, wobei der Alkylsubstituent im alkylsubstituierten Arylrest
               verzweigt und gesättigt
                  oder
               unverzweigt und gesättigt ist,
   wobei
   - b: 0, 1 oder 2 bedeutet und
   - a: 3-b bedeutet
   und
(B) ein, zwei, drei oder mehr als drei zweite, strukturell jeweils identische Siloxaneinheiten, die von der ersten Siloxaneinheit strukturell verschieden sind.

Die vorliegende Erfindung betrifft außerdem eine Polysiloxanverbindung (vorzugsweise wie vorstehend definiert, besonders bevorzugt wie vorstehend als bevorzugt definiert), umfassend
(A) ein, zwei, drei oder mehr als drei strukturell jeweils identische erste Siloxaneinheiten ausgewählt aus der Gruppe bestehend aus Siloxaneinheiten der allgemeinen Formel (Ib) 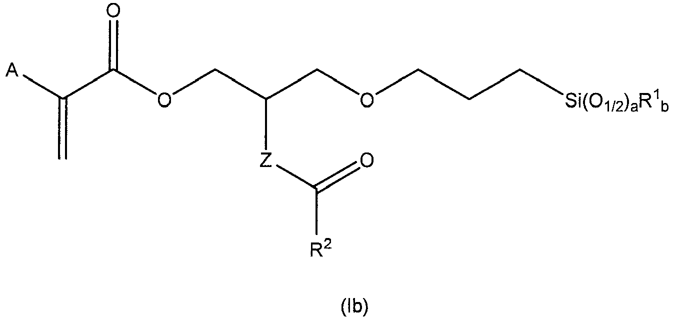 wobei für die Struktureinheiten A, Z, R¹ und R² in jeder der strukturell identischen ersten Siloxaneinheiten unabhängig voneinander gilt:
   A bedeutet H oder CH₃,
   R¹ bedeutet einen Alkylrest mit insgesamt 1 bis 4 Kohlenstoffatomen,
   Z bedeutet O, S oder NH, vorzugsweise O
      und
   R² ist ausgewählt aus der Gruppe bestehend aus
      verzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
      unverzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
      unsubstituierter oder alkylsubstituierter Arylrest mit insgesamt 9 bis 18 Kohlenstoffatomen, vorzugsweise 10 bis 18 Kohlenstoffatomen, besonders bevorzugt 10 bis 14 Kohlenstoffatomen, wobei der Alkylsubstituent im alkylsubstituierten Arylrest
         verzweigt und gesättigt
            oder
         unverzweigt und gesättigt ist,
   wobei
   - b: 0, 1 oder 2 bedeutet und
   - a: 3-b bedeutet
   und
(B) ein, zwei, drei oder mehr als drei zweite, strukturell jeweils identische Siloxaneinheiten, die von der ersten Siloxaneinheit strukturell verschieden sind.

Besonders bevorzugt ist eine erfindungsgemäße Polysiloxanverbindung (wie vorstehend definiert, vorzugsweise wie vorstehend als bevorzugt definiert), umfassend
(A) ein, zwei, drei oder mehr als drei strukturell jeweils identische erste Siloxaneinheiten ausgewählt aus der Gruppe bestehend aus Siloxaneinheiten der allgemeinen Formel (Ic) 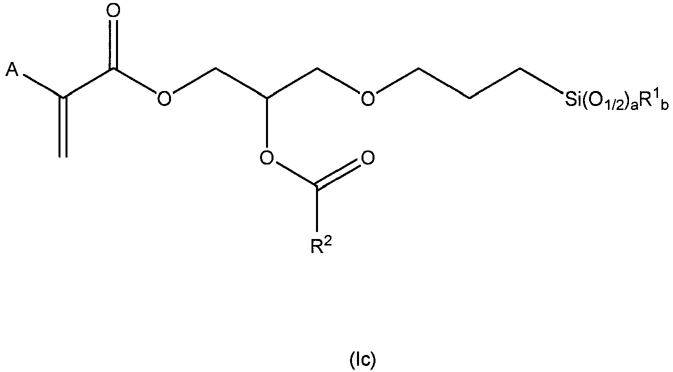 wobei für die Struktureinheiten A, R¹ und R² in jeder der strukturell identischen ersten Siloxaneinheiten unabhängig voneinander gilt:
   A bedeutet H oder CH₃,
   R¹ bedeutet einen Alkylrest mit insgesamt 1 bis 4 Kohlenstoffatomen, und
   R² ist ausgewählt aus der Gruppe bestehend aus
      verzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
      unverzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
      unsubstituierter oder alkylsubstituierter Arylrest mit insgesamt 9 bis 18 Kohlenstoffatomen, vorzugsweise 10 bis 18 Kohlenstoffatomen, besonders bevorzugt 10 bis 14 Kohlenstoffatomen, wobei der Alkylsubstituent im alkylsubstituierten Arylrest
         verzweigt und gesättigt
            oder
         unverzweigt und gesättigt ist,
      wobei
      - b: 0, 1 oder 2 bedeutet und
      - a: 3-b bedeutet
      und
(B) ein, zwei, drei oder mehr als drei zweite, strukturell jeweils identische Siloxaneinheiten, die von der ersten Siloxaneinheit strukturell verschieden sind.

Ganz besonders bevorzugt ist eine erfindungsgemäße Polysiloxanverbindung (wie vorstehend definiert, vorzugsweise wie vorstehend als bevorzugt definiert), wobei R² ausgewählt ist aus der Gruppe bestehend aus 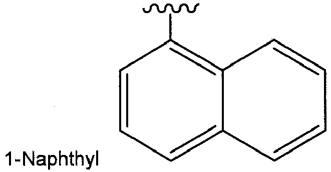 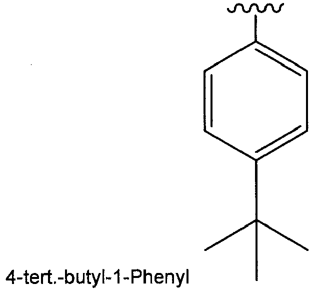 und

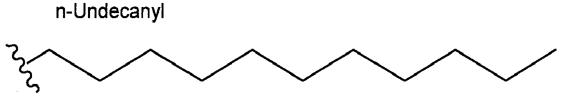

Die in den vorstehend abgebildeten Strukturformeln gezeichneten Schlängellinien kennzeichnen jeweils die R² mit dem benachbarten Kohlenstoffatom der Gruppe Z-(CO)-R² der erfindungsgemäßen Polysiloxanverbindung verknüpfende chemische Bindung.

Der Begriff n-Undecanyl bedeutet ein Alkylradikal (abgeleitet von dem Alkan n-Undekan) mit der Formel CH₃(CH₂)₉CH₂-, wobei der Bindestrich in der Formel die verknüpfende chemische Bindung bedeutet. Im vorliegenden Text ist der Begriff n-Undecanyl gleichbedeutend mit dem Begriff n-Undecyl.

Insbesondere bevorzugt ist eine erfindungsgemäße Polysiloxanverbindung (wie vorstehend definiert, vorzugsweise wie vorstehend als bevorzugt definiert), umfassend
(A) ein, zwei, drei oder mehr als drei strukturell jeweils identische erste Siloxaneinheiten ausgewählt aus der Gruppe bestehend aus:

| Nr. | Siloxaneinheit | A | R¹ | R² |
|---|---|---|---|---|
| (1) | 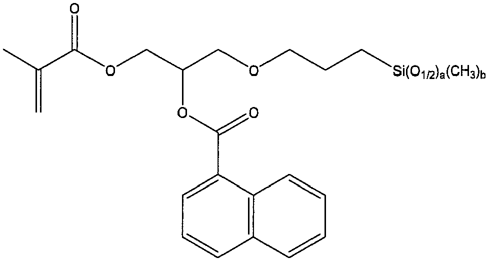 | CH₃ | CH₃ | 1-Naphtyl |
| (2) | 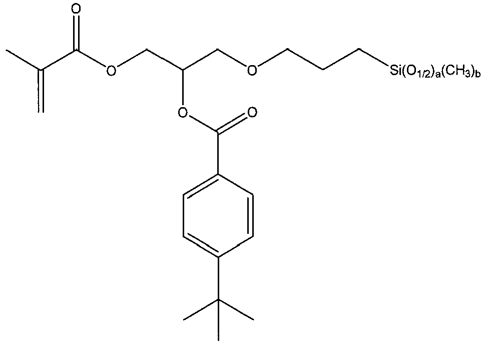 | CH₃ | CH₃ | 4-tert.-butyl-1-Phenyl |
| (3) | 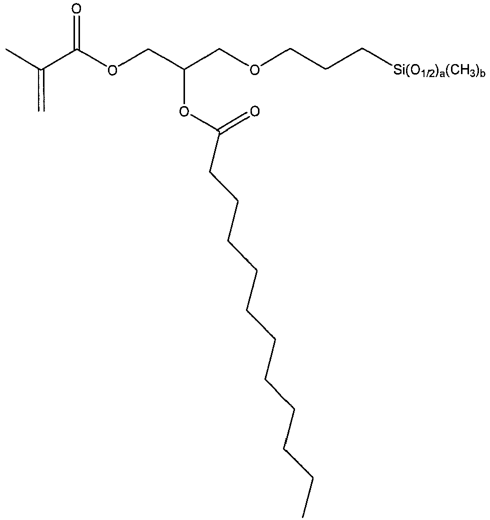 | CH₃ | CH₃ | n-Undecanyl |
| (4) | 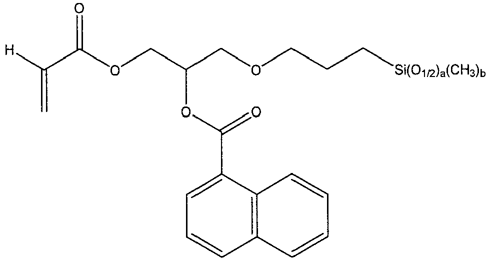 | H | CH₃ | 1-Naphtyl |
| (5) | 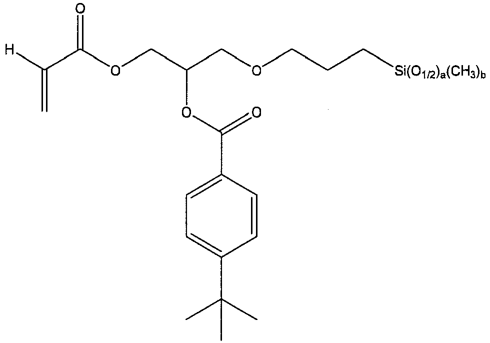 | H | CH₃ | 4-tert.-butyl-1-Phenyl |
| (6) | 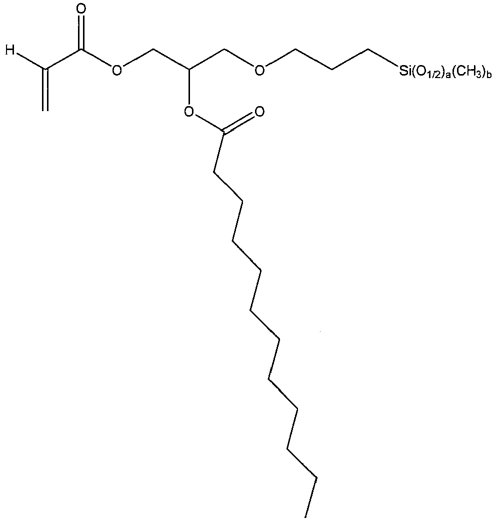 | H | CH₃ | n-Undecanyl |
| (7) | 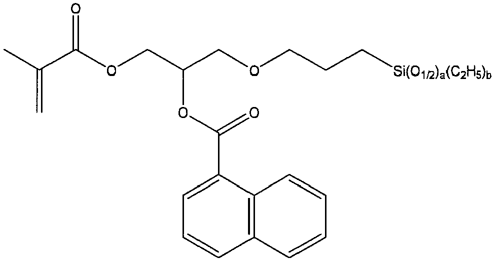 | CH₃ | C₂H₅ | 1-Naphtyl |
| (8) | 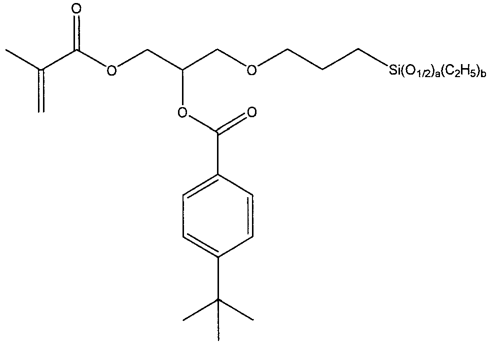 | CH₃ | C₂H₅ | 4-tert.-butyl-1-Phenyl |
| (9) | 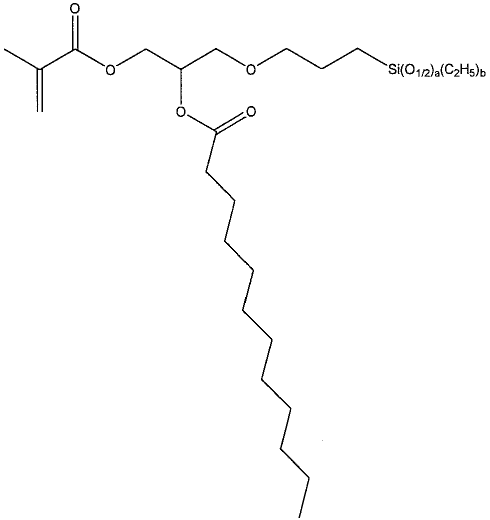 | CH₃ | C₂H₅ | n-Undecanyl |
| (10) | 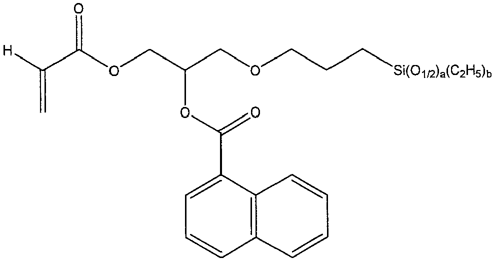 | H | C₂H₅ | 1-Naphtyl |
| (11) | 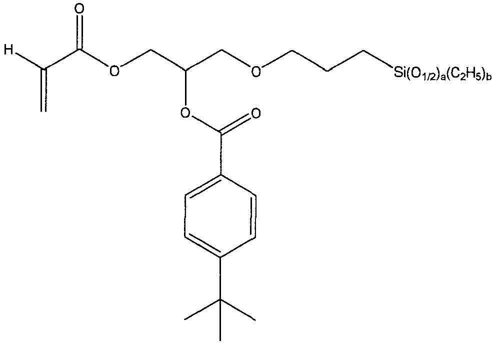 | H | C₂H₅ | 4-tert.-butyl-1-Phenyl |
| (12) | 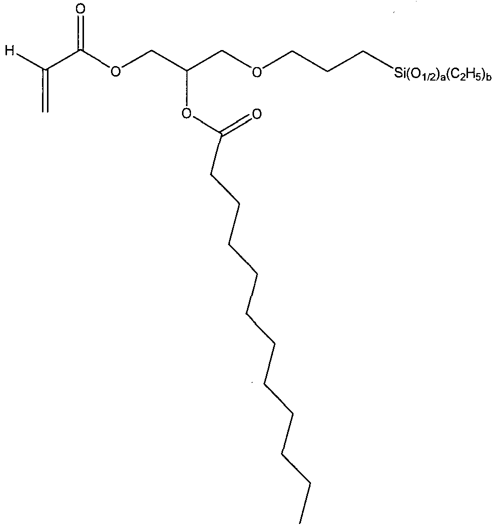 | H | C₂H₅ | n-Undecanyl |
| (13) | 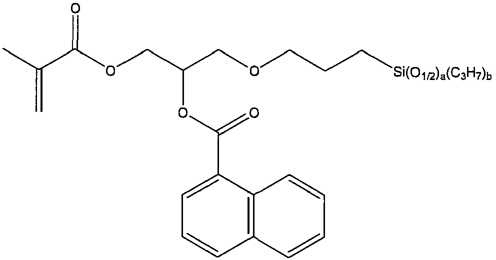 | CH₃ | C₃H₇ | 1-Naphtyl |
| (14) | 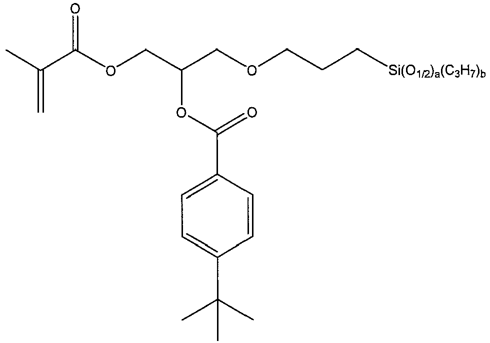 | CH₃ | C₃H₇ | 4-tert.-butyl-1-Phenyl |
| (15) | 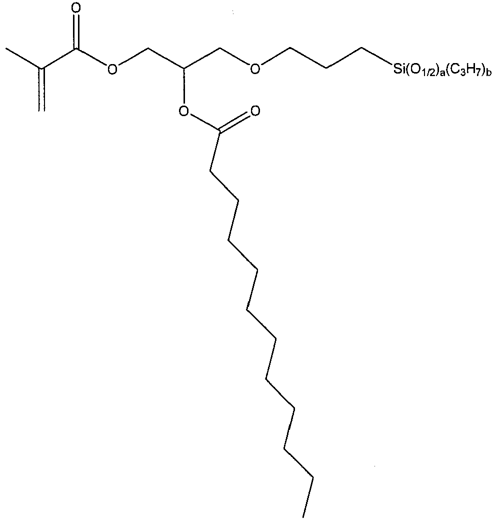 | CH₃ | C₃H₇ | n-Undecanyl |
| (16) | 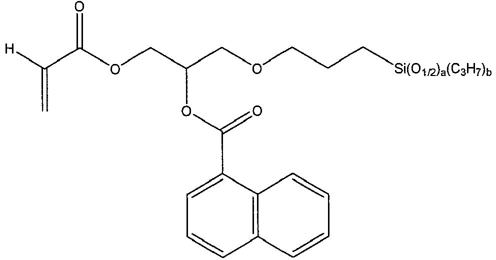 | H | C₃H₇ | 1-Naphtyl |
| (17) | 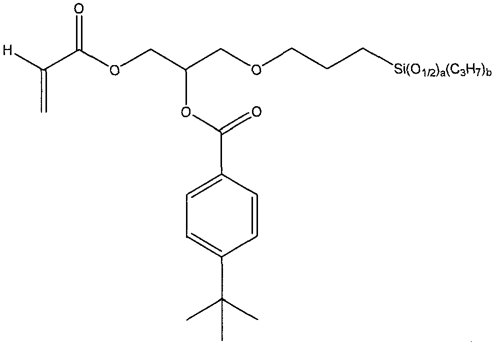 | H | C₃H₇ | 4-tert.-butyl-1-Phenyl |
| (18) | 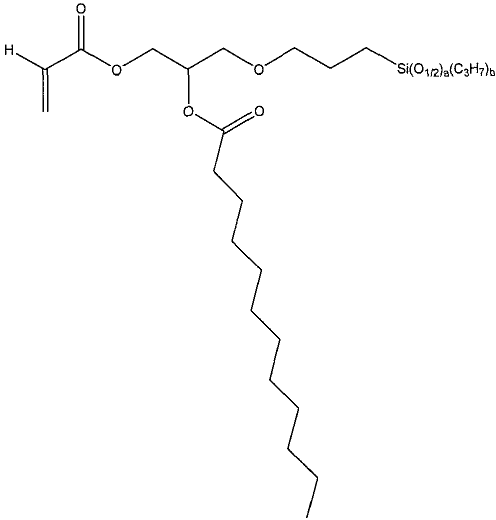 | H | C₃H₇ | n-Undecanyl |
| (19) | 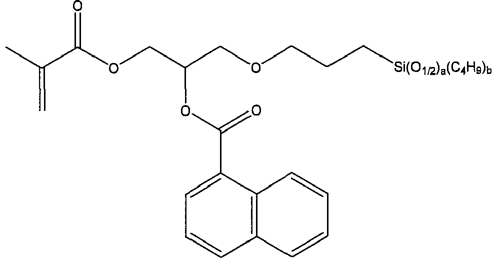 | CH₃ | C₄H₉ | 1-Naphtyl |
| (20) | 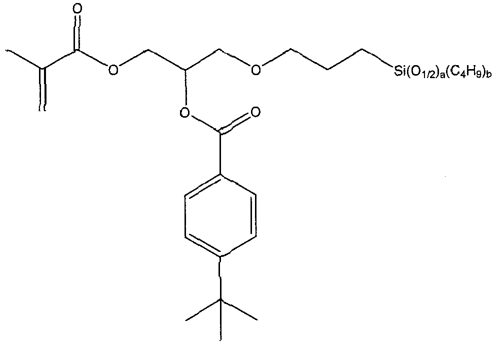 | CH₃ | C₄H₉ | 4-tert.-butyl-1-Phenyl |
| (21) | 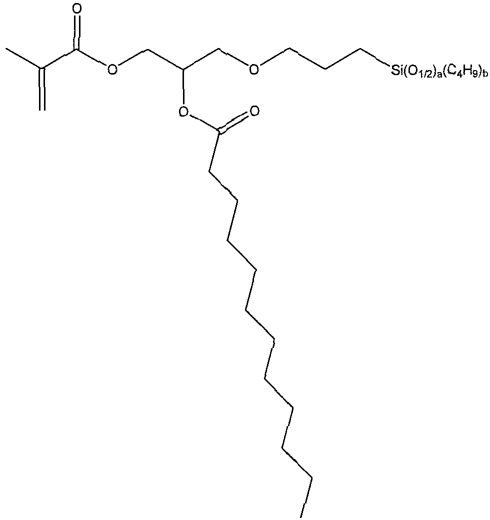 | CH₃ | C₄H₉ | n-Undecanyl |
| (22) | 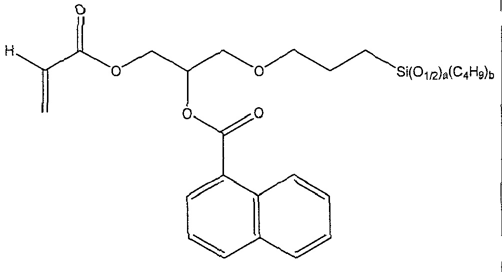 | | | 1-Naphtyl |
| (23) | 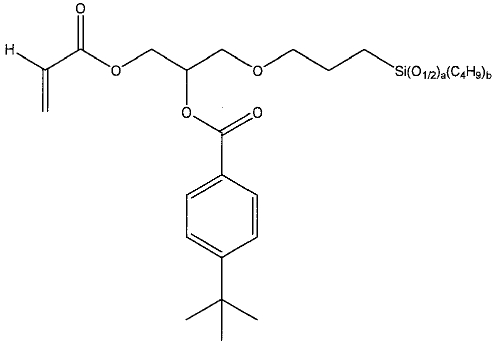 | H | C₄H₉ | 4-tert.-butyl-1-Phenyl |
| (24) | 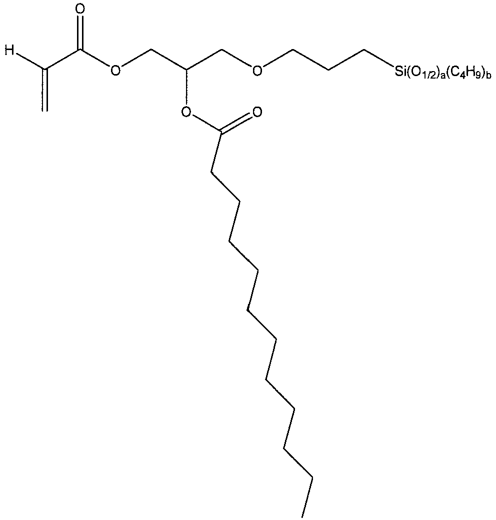 | H | C₄H₉ | n-Undecanyl |

wobei
- b: 0, 1 oder 2 bedeutet und
- a: 3-b bedeutet.

Die vorliegende Erfindung betrifft auch Mischungen umfassend zwei, drei oder mehr als drei unterschiedliche erfindungsgemäße Polysiloxanverbindungen (wie vorstehend definiert, vorzugsweise wie vorstehend als bevorzugt definiert). Besonders bevorzugt betrifft die vorliegende Erfindung Mischungen umfassend zwei, drei oder mehr als drei unterschiedliche erfindungsgemäße Polysiloxanverbindungen (wie vorstehend definiert, vorzugsweise wie vorstehend als bevorzugt definiert) zur Anwendung in einem therapeutischen Verfahren (einem Verfahren zur therapeutischen Behandlung des menschlichen oder tierischen Körpers, vorzugsweise des menschlichen Körpers), vorzugsweise als polymerisierbarer Bestandteil eines härtbaren Dentalmaterials. Ganz besonders bevorzugt betrifft die vorliegende Erfindung Mischungen umfassend zwei, drei oder mehr als drei unterschiedliche erfindungsgemäße Polysiloxanverbindungen (wie vorstehend definiert, vorzugsweise wie vorstehend als bevorzugt definiert) zur spezifischen Anwendung in einem therapeutischen Verfahren (einem Verfahren zur therapeutischen Behandlung des menschlichen oder tierischen Körpers, vorzugsweise des menschlichen Körpers) zum temporären oder permanenten Füllen einer dentalen Kavität
oder
in einem therapeutischen Verfahren (einem Verfahren zur therapeutischen Behandlung des menschlichen oder tierischen Körpers, vorzugsweise des menschlichen Körpers) als
dentales Füllungsmaterial,
dentales Unterfüllungsmaterial,
dentales Adhäsiv (Bonding),
als fliessfähiges Kompositmaterial (Flow-Material),
als Fissurenversiegler,
als Kronenmaterial,
als Inlay und/oder Onlay,
als Brückenmaterial
und/oder als Stumpfaufbaumaterial.

Die vorliegende Erfindung betrifft auch ein härtbares Dentalmaterial umfassend eine oder mehr als eine erfindungsgemäße Polysiloxanverbindung (wie vorstehend definiert, vorzugsweise wie vorstehend als bevorzugt definiert) und eine oder mehr als eine Verbindung, die keine erfindungsgemäße Polysiloxanverbindung (wie vorstehend definiert, vorzugsweise wie vorstehend als bevorzugt definiert) ist. Weitere Details zu Verbindungen, die keine erfindungsgemäßen Polysiloxanverbindungen sind, siehe unten im vorliegenden Text. Das in Verbindung mit erfindungsgemäßen Polysiloxanverbindungen Gesagte gilt für das erfindungsgemäße härtbare Dentalmaterial entsprechend.

In bevorzugten Fällen umfasst das erfindungsgemäße härtbare Dentalmaterial eine erfindungsgemäße Mischung (wie vorstehend definiert, vorzugsweise wie vorstehend als bevorzugt definiert) umfassend zwei, drei oder mehr als drei unterschiedliche erfindungsgemäße Polysiloxanverbindungen (wie vorstehend definiert, vorzugsweise wie vorstehend als bevorzugt definiert).

Bevorzugt ist ein härtbares Dentalmaterial (wie vorstehend beschrieben), weiter umfassend ein, zwei, mehr als zwei oder sämtliche Substanzen aus der Gruppe bestehend aus:
- dentale organische Füllstoffpartikel, die vorzugsweise röntgenopak und/oder nanoskalig sind,
- dentale anorganische Füllstoffpartikel, die vorzugsweise röntgenopak und/oder nanoskalig sind,
- dentale organisch oberflächenmodifizierte anorganische Füllstoffpartikel, die vorzugsweise röntgenopak und/oder nanoskalig sind,
- rheologische Hilfsmittel,
- Polymerisationsinitiatoren, vorzugsweise Photoinitiatoren,
- chemische Verbindungen als Katalysatoren bzw. Bestandteile von Katalysatorsystemen,
- Farbmittel, vorzugsweise Farbpigmente,
- Stabilisatoren, insbesondere Tageslichtstabilisatoren,
- Inhibitoren,
- Aktivatoren,
- Molekulargewichtsregler,
- Konservierungsmittel,
- grenzflächenaktive Substanzen,
- Mikrobizide, vorzugsweise Bakterizide,
- organische, vorzugsweise radikalisch polymerisierbare Monomere, die keine erfindungsgemäßen Polysiloxane sind, vorzugsweise zur Umsetzung mit der erfindungsgemäßen Polysiloxanverbindung,
- organische Polymere und Oligomere und Verbindungen mit hohen Molekulargewichten, vorzugsweise Weichmacher,
- Verdickungsmittel und
- dentale Arzneimittel.

Bevorzugte dentale Füllstoffpartikel für eine erfindungsgemäße härtbare Dentalmischung sind organische Füllstoffpartikel oder anorganische Füllstoffpartikel oder Mischungen aus organischen und anorganischen Füllstoffpartikeln. Besonders bevorzugt sind anorganische Füllstoffpartikel, vorzugsweise röntgenopake Füllstoffpartikel.

Die dentalen anorganischen Füllstoffpartikel können sämtlich chemisch identisch sein oder als Mischungen chemisch verschiedener dentaler anorganischer Füllstoffpartikel eingesetzt werden.

Die dentalen anorganischen Füllstoffpartikel sind vorzugsweise ausgewählt aus der Gruppe bestehend aus Mischoxiden aus SiO₂, ZrO₂ und/oder TiO₂, pyrogene Kieselsäure, Fällungskieselsäure, Bariumsilikatgläser, Bariumfluorsilikatgläser, Strontiumsilikatgläser, Strontiumborsilikatgläser, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate und schwerlösliche Metallsalze wie Bariumsulfat oder Calciumfluorid. Bevorzugt eingesetzte röntgenopake Füllstoffpartikel sind regelmäßig anorganische Füllstoffpartikel und sind vorzugsweise ausgewählt aus der Gruppe bestehend aus Fluoriden der Selten-Erdmetalle (wie Ytterbiumfluorid und Yttriumtrifluorid) und Strontiumhexafluorozirkonat. Ein besonders bevorzugter röntgenopaker anorganischer Füllstoff ist Ytterbiumfluorid. Für bestimmte Anwendungen ist eine Mischung aus röntgenopaken Füllstoffpartikeln (vorzugsweise wie vorstehend beschrieben) und anorganischen Füllstoffpartikeln, die selbst nicht zu einer Röntgenopazität beitragen, bevorzugt. Besonders bevorzugt sind hier Bariumsilikatgläser und/oder Bariumfluoridsilikatgläser.

In einem erfindungsgemäßen härtbaren Dentalmaterial werden besonders bevorzugt dentale Füllstoffpartikel eingesetzt, die einen Brechungsindex im Bereich von 1,46 bis 1,56 besitzen, vorzugsweise im Bereich von 1,48 bis 1,54
und/oder für die gilt:
der Betrag der Differenz des Brechungsindexes aus
   - Brechungsindex der Gesamtheit der dentalen Füllstoffpartikel und Brechungsindex der Gesamtheit an der oder den erfindungsgemäßen Polysiloxanverbindungen
liegt im Bereich von 0,01 bis 0,04.

Besitzt beispielsweise die Gesamtheit an der oder den erfindungsgemäßen Polysiloxanverbindungen einen Brechungsindex von 1,50 und die Gesamtheit der dentalen Füllstoffpartikel einen Brechungsindex von 1,51, beträgt die Differenz 0,01.

Bevorzugt ist der Einsatz oberflächenmodifizierter anorganischer Füllstoffpartikel. Bevorzugt einzusetzende oberflächenmodifizierte anorganische Füllstoffpartikel werden durch Oberflächenmodifizierung mit einem Silan wie Methacryloxypropyltrimethoxysilan erhalten.

Organische Füllstoffpartikel umfassen oder bestehen aus beispielsweise ein oder mehr Verbindungen ausgewählt aus der Gruppe bestehend aus Polyvinylacetat und Copolymere des Polyvinylacetats mit einer oder mehreren polymerisierbaren Verbindungen, Polystyrol, Polyethylen, Polypropylen, Wachse wie Polyethylenwachs, Polybutylen, Polybutadien, Copolymere des Butadiens und des Styrols, Polyacrylnitril, Harze wie Kolophoniumharz oder Kohlenwasserstoffharze, Poly(meth)acrylatester, d.h. Umsetzungsprodukte von Poly(meth)acrylsäure mit linearen oder verzweigten aliphatischen, aromatischen oder cycloaliphatischen Alkoholen wie Methanol, Ethanol, Propanol, Isopropanol, den isomeren Butanolen und höheren Homologen der genannten Alkohole mit bis zu 22 Kohlenstoffatomen, Cyclohexanol, Benzylalkohol und dergleichen, Polydialkylmaleinate wie Dibutylmaleinat und deren Copolymere und Silylgruppen-haltige Polymere wie Polyvinylsilane oder Copolymere von Vinylsilan mit einem oder mehreren der genannten Monomeren.

Bevorzugte erfindungsgemäße härtbare Dentalmaterialien (wie vorstehend definiert, vorzugsweise wie vorstehend als bevorzugt definiert) sind lichthärtbar (photohärtbar). Die Vernetzung/Polymerisation der erfindungsgemäßen Polysiloxanverbindung in solchen erfindungsgemäßen Dentalmaterialien erfolgt dann regelmäßig durch Einwirkung von Licht bestimmter Wellenlängen und in Gegenwart von Photoinitiatoren. Beispiele für einen Photoinitiator schließen Verbindungen ein, die nur photosensibilisierend wirken, sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Beispiele der unterschiedlichen Klassen finden sich beispielsweise in DE 10 2006 019 092 A1 und DE 39 41 629 C2, welche im Wege der Verweisung Bestandteil der vorliegenden Anmeldung sind.

Beispiele für Beschleuniger, die zusammen mit Sensibilisatoren eingesetzt werden, sind tertiäre Amine, sekundäre Amine, Barbitursäuren, Zinnverbindungen, Aldehyde und Schwefelverbindungen. Konkrete Beispiele der unterschiedlichen Klassen finden sich in DE 10 2006 019 092 und DE 39 41 629 C2, welche im Wege der Verweisung Bestandteil der vorliegenden Anmeldung sind.

Weitere geeignete Initiatoren sowie Initiatorkombinationen sind in DE 601 16 142 T2 beschrieben, welche im Wege der Verweisung Bestandteil der vorliegenden Anmeldung ist.

Im Rahmen der vorliegenden Erfindung bevorzugt verwendeten Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung (Vernetzung/Polymerisation) eines erfindungsgemäßen bzw. erfindungsgemäß anzuwendenden bzw. einzusetzenden härtbaren Dentalmaterials bewirken können.

Weitere bevorzugte erfindungsgemäße härtbare Dentalmaterialien (wie vorstehend definiert, vorzugsweise wie vorstehend als bevorzugt definiert) sind durch chemische Aushärtung härtbar. Hierzu sind dem Fachmann diverse Initiatoren für eine chemische Aushärtung bekannt. Es sei insoweit exemplarisch auf die Offenbarung in EP 1 720 506 A1 verwiesen.

In vielen Fällen ist ein erfindungsgemäßes härtbares Dentalmaterial bevorzugt, welches sowohl lichthärtbar ist als auch chemisch härtbar ist. Diese bevorzugten dualhärtenden Dentalmaterialien umfassen eine Kombination aus Photoinitiatoren und Initiatoren zur chemischen Aushärtung. Die vorstehenden Ausführungen zu bevorzugten Initiatoren gelten entsprechend.

Bevorzugte erfindungsgemäße lichthärtbare Dentalmaterialien (wie vorstehend definiert, vorzugsweise wie vorstehend als bevorzugt definiert, einschließlich der dualhärtenden erfindungsgemäßen Dentalmaterialien), enthalten vorzugsweise einen oder mehrere Inhibitoren (auch Stabilisatoren genannt). Diese werden üblicherweise zugesetzt, um eine Spontanpolymerisation zu vermeiden. Sie reagieren mit vorzeitig entstehenden Radikalen, die abgefangen werden, so dass eine vorzeitige Polymerisation verhindert wird. Das erhöht die Lagerstabilität der bevorzugten lichthärtbaren Dentalmaterialen (bzw. der dualhärtenden Dentalmaterialien). Bevorzugt einzusetzende Inhibitoren sind Phenolderivate wie Hydrochinonmonomethylether (HQME) oder 2,6-Di-tert.butyl-4-methylphenol (BHT). Weitere bevorzugt einzusetzende Inhibitoren wie tert.-Butylhydroxyanisol (BHA), 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in EP 0 783 880 B1 beschrieben, welche im Wege der Verweisung Bestandteil der vorliegenden Anmeldung ist. Alternative bevorzugte Inhibitoren sind in DE 101 19 831 A1 und in EP 1 563 821 A1 angegeben, welche im Wege der Verweisung Bestandteil der vorliegenden Anmeldung sind.

Bevorzugte erfindungsgemäße lichthärtbare Dentalmaterialien (wie vorstehend definiert, vorzugsweise wie vorstehend als bevorzugt definiert, einschließlich der dualhärtenden erfindungsgemäßen Dentalmaterialien), enthalten vorzugsweise einen oder mehrere organische, vorzugsweise radikalisch polymerisierbare Monomere, insbesondere monofunktionelle und/oder polyfunktionelle Methacrylate, die allein oder in Mischungen eingesetzt werden können. Beispiele für diese Verbindungen sind Alkylmethacrylate wie beispielsweise Methylmethacrylat, Ethylmethacrylat, Propylmethacrylat, Butylmethacrylat, etc., Alkylenglykoldimethacrylate und Polyethylenglykoldimethacrylate wie beispielsweise Tetraethylenglycoldimethacrylat, Triethylenglycoldimethacrylat, Diethylenglycoldimethacrylat, Ethylenglycoldimethacrylat, Butandioldimethacrylat, Hexandioldimethacrylat, Decandioldimethacrylat, Dodecandioldimethacrylat, die Mono-, Di,- Tri-, Tetra- oder Pentamethacrylate mehrwertiger Alkohole wie beispielsweise Trimethylolpropandimethacrylat, Dipentaerythritolpentamethacrylat, Dimethylolpropantrimethacrylat, Neopentylglykolmethacrylat, die Methacrylate von alkoxyliertem, (ethoxyliertem, propoxyliertem, etc.) Glycerin, Trimethylolpropan, Pentaerythrit, Dipentaerythrit, Dimethylolpropan, etc. sowie deren technische Gemische, Bisphenol-A-dimethacrylat, 2,2-Bis-4(3-methacryloxy-2-hydroxypropoxy)-phenylpropan (Bis-GMA) sowie die Reaktionsprodukte aus Isocyanaten, insbesondere Di- und/oder Triisocyanaten und OH-gruppenhaltigen Methacrylaten sowie die Methacrylate der radikalisch polymerisierbaren TCD (Tricyclodecan)-derivate wie TCD-di-HEMA und/oder TCD di-HEA sowie polymerisierbare Verbindungen, wie sie aus der EP 2 436 668 B1 bekannt sind.

Bevorzugte erfindungsgemäße härtbare Dentalmaterialien (wie vorstehend definiert, vorzugsweise wie vorstehend als bevorzugt definiert) besitzen charakteristische Farben, vorzugsweise eine Zahnfarbe, die von der "VITA classical A1 - D4 Farbskala" umfasst ist; solche Farben werden bezeichnet mit A1 - A4 (Rötlichbräunlich), B1 - B4 (Rötlich-gelblich), C1 - C4 (Grautöne), D2 - D4 (Rötlich-grau). Bevorzugte Farben können mittels Farbmitteln, vorzugsweise Farbpigmenten, eingestellt werden.

Wie bereits oben beschrieben, kann das erfindungsgemäße härtbare Dentalmaterial gehärtet werden. Die vorliegende Erfindung betrifft somit auch ein gehärtetes Dentalmaterial, erhältlich aus einem erfindungsgemäßen härtbaren Dentalmaterial (wie vorstehend definiert, vorzugsweise ein härtbares Dentalmaterial wie vorstehend als bevorzugt definiert) mittels Polymerisation der im Dentalmaterial enthaltenen Polysiloxanverbindung sowie gegebenenfalls weiterer im Dentalmaterial enthaltener polymerisierbarer Bestandteile. Die Polymerisation bzw. Vernetzung erfolgt mittels der in den (Meth)acrylatgruppen enthaltenen organisch polymerisierbaren Doppelbindungen.

Das zu bevorzugten Ausführungsformen erfindungsgemäßer Polysiloxanverbindungen und erfindungsgemäßer härtbarer Dentalmaterialien Gesagte gilt für die erfindungsgemäßen gehärteten Dentalmaterialien entsprechend.

Ein wesentlicher Aspekt der vorliegenden Erfindung betrifft eine erfindungsgemäße Polysiloxanverbindung (wie vorstehend definiert, vorzugsweise eine Polysiloxanverbindung wie vorstehend als bevorzugt definiert) bzw. ein erfindungsgemäßes härtbares Dentalmaterial (wie vorstehend definiert, vorzugsweise ein härtbares Dentalmaterial wie vorstehend als bevorzugt definiert) bzw. ein erfindungsgemäßes gehärtetes Dentalmaterial (wie vorstehend definiert, vorzugsweise ein gehärtetes Dentalmaterial wie vorstehend als bevorzugt definiert) zur Anwendung in einem therapeutischen Verfahren (einem Verfahren zur therapeutischen Behandlung des menschlichen oder tierischen Körpers, vorzugsweise des menschlichen Körpers), wobei eine erfindungsgemäße Polysiloxanverbindung vorzugsweise als polymerisierbarer Bestandteil eines härtbaren Dentalmaterials eingesetzt wird.
Besonders bevorzugt ist insoweit die spezifische Anwendung der erfindungsgemäßen Polysiloxanverbindung (Polysiloxanverbindungen wie vorstehend definiert, vorzugsweise Polysiloxanverbindungen wie vorstehend als bevorzugt definiert) bzw. des erfindungsgemäßen härtbaren Dentalmaterials (härtbare Dentalmaterialen wie vorstehend definiert, vorzugsweise härtbare Dentalmaterialen wie vorstehend als bevorzugt definiert) bzw. des erfindungsgemäßen gehärteten Dentalmaterials (wie vorstehend definiert, vorzugsweise ein gehärtetes Dentalmaterial wie vorstehend als bevorzugt definiert) in einem therapeutischen Verfahren (einem Verfahren zur therapeutischen Behandlung des menschlichen oder tierischen Körpers, vorzugsweise des menschlichen Körpers) zum temporären oder permanenten Füllen einer dentalen Kavität
oder
in einem therapeutischen Verfahren (einem Verfahren zur therapeutischen Behandlung des menschlichen oder tierischen Körpers, vorzugsweise des menschlichen Körpers) als
dentales Füllungsmaterial,
dentales Unterfüllungsmaterial,
dentales Adhäsiv (Bonding),
als fliessfähiges Kompositmaterial (Flow-Material),
als Fissurenversiegler,
als Kronenmaterial,
als Inlay und/oder Onlay,
als Brückenmaterial
und/oder als Stumpfaufbaumaterial.

In einigen bevorzugten therapeutischen Verfahren (Verfahren zur therapeutischen Behandlung des menschlichen oder tierischen Körpers, vorzugsweise des menschlichen Körpers) wird das erfindungsgemäße härtbare Dentalmaterial als Fissurenversiegler und/oder zum Versiegeln kariöser Läsionen angewendet. In diesen und anderen Fällen umfasst das erfindungsgemäße härtbare Dentalmaterial vorzugsweise eine oder mehrere fluoridabgebende Substanzen, vorzugsweise Substanzen, die Natriumfluorid und/oder Aminfluoride abgeben.

Eigene Untersuchungen haben gezeigt, dass die erfindungsgemäßen Polysiloxanverbindungen (wie vorstehend definiert, vorzugsweise Polysiloxanverbindungen wie vorstehend als bevorzugt definiert) und die erfindungsgemäßen härtbaren Dentalmaterialien (wie vorstehend definiert, vorzugsweise härtbare Dentalmaterialien wie vorstehend als bevorzugt definiert) zu hervorragenden Ergebnissen bei den vorstehend genannten spezifischen Anwendungen führen.

Die vorliegende Erfindung betrifft außerdem ein Verfahren zur Herstellung einer Polysiloxanverbindung (wie vorstehend definiert, vorzugsweise Polysiloxanverbindungen wie vorstehend als bevorzugt definiert), mit den folgenden Schritten:
- Herstellen oder Bereitstellen einer intermediären Polysiloxanverbindung umfassend
   - (A_{P}) ein, zwei, drei oder mehr als drei strukturell jeweils identische erste intermediäre Siloxaneinheiten ausgewählt aus der Gruppe bestehend aus Siloxaneinheiten der allgemeinen Formel (I_{P}) 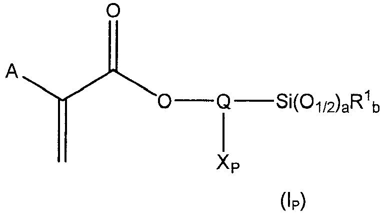
      wobei für die Struktureinheiten A, Q, X_{P} und R¹ in jeder der strukturell identischen ersten Siloxaneinheiten unabhängig voneinander gilt:
      A bedeutet H oder CH₃,
      Q bedeutet eine den Substituenten X_{P} tragende Verknüpfungsgruppe,
      R¹ bedeutet einen Alkylrest mit insgesamt 1 bis 4 Kohlenstoffatomen und
      X_{P} eine reaktive Gruppe
      wobei
         - b: 0, 1 oder 2 bedeutet und
         - a: 3-b bedeutet,
- Umsetzen der intermediären Polysiloxanverbindung in ein oder mehr Schritten unter Reaktion der reaktiven Gruppe X_{P}, so dass die erfindungsgemäße Polysiloxanverbindung gebildet wird (eine Polysiloxanverbindung wie vorstehend definiert, vorzugsweise eine Polysiloxanverbindung wie vorstehend als bevorzugt definiert).

Verfahren zur Herstellung einer intermediären Polysiloxanverbindung sind dem Fachmann bekannt. Insbesondere erfolgt die Herstellung über Kondensationsreaktionen vorhandener bzw. vormals synthetisierter Silane (siehe z.B. DE 4416857 C1). Dabei wird in einem ersten Schritt zunächst die monomere Silaneinheit hergestellt (vgl. das exemplarische Schema (c), wobei A CH₃ oder H bedeutet, Edukt 1: (Meth)acrylsäure, Edukt 2: 3-Glycidyloxypropylmethyldiethoxysilan, Reaktionsprodukt: monomere Silaneinheit). Die reaktive Gruppe in der monomeren Silaneinheit ist häufig eine Hydroxygruppe.

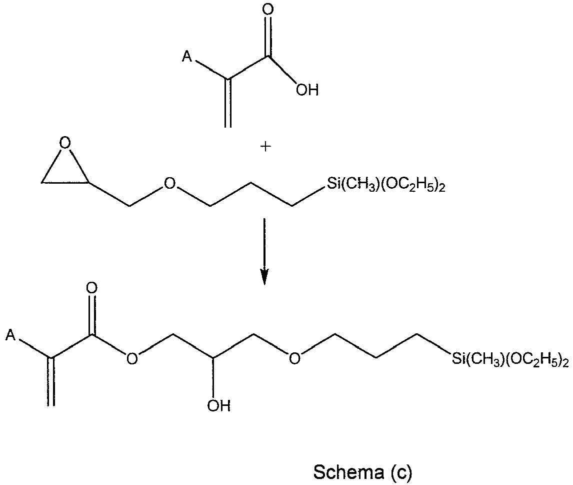

Die hergestellten monomeren Silaneinheiten werden anschließend kondensiert, um eine intermediäre Polysiloxanverbindung zu erhalten (vgl. das schematische und exemplarische Schema (d)), wobei A CH₃ oder H bedeutet und O_{1/2} für die Sauerstoffverknüpfung zum nächsten Siliziumatom in der intermediären Polysiloxanverbindung steht).

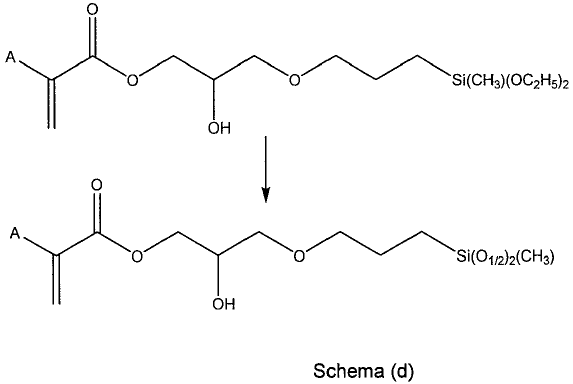

Gemäß dem exemplarischen Schema (c) hergestellte monomere Silaneinheiten können auch mit weiteren, strukturell verschieden monomeren Silaneinheiten umgesetzt (cokondensiert) werden.

Ein gemäß dem exemplarischen Schema (d) gebildetes Reaktionsprodukt ist eine intermediäre Polysiloxanverbindung im Sinne des vorliegenden Textes und besitzt eine oder mehrere freie polare Hydroxygruppen (reaktive Hydroxygruppen) als exemplarische reaktive Gruppe. Diese reaktiven Hydroxygruppen werden in einem anschließenden Reaktionsschritt z.B. mit Carbonsäurederivate umgesetzt, so dass erfindungsgemäße Polysiloxanverbindungen erhalten werden (vgl. die nachfolgenden exemplarischen Schemata (f), (g) und (h), wobei jeweils A CH₃ oder H bedeutet und O_{1/2} für die Sauerstoffverknüpfung zum nächsten Siliziumatom in den erfindungsgemäßen Polysiloxanverbindungen steht). In den Schemata (f), (g) und (h) ist exemplarisch die Umsetzung eines Reaktionsprodukts (intermediäre Polysiloxanverbindung) gemäß exemplarischem Schema (d) mit verschiedenen Säurechloridverbindungen gezeigt.

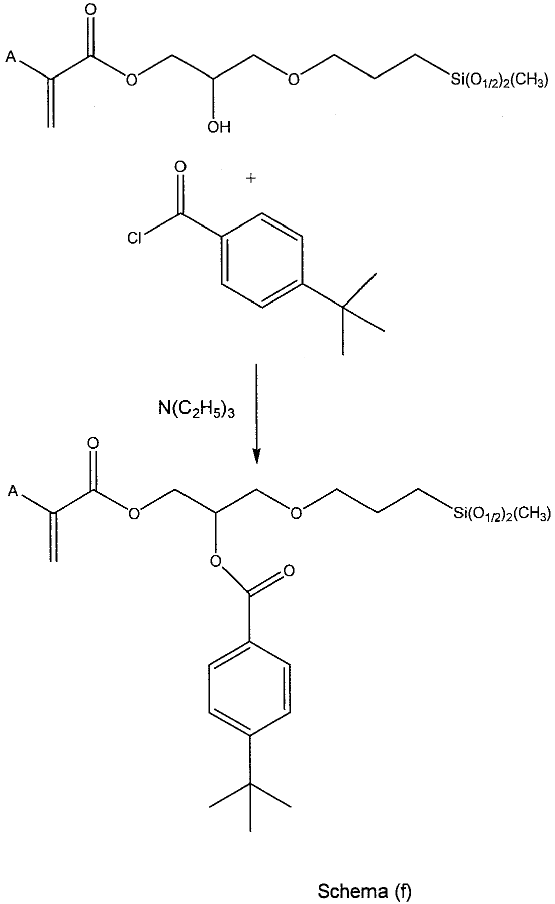

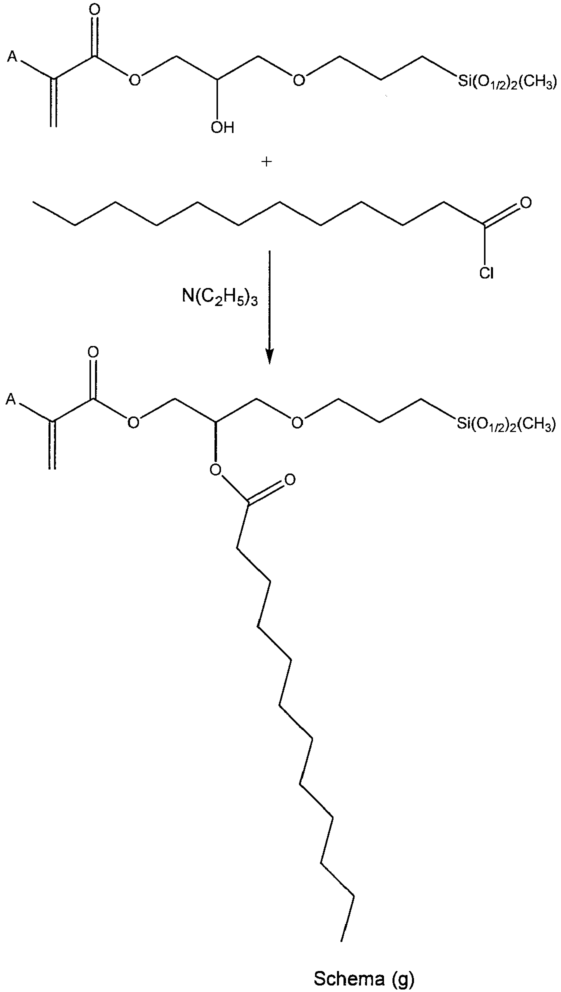

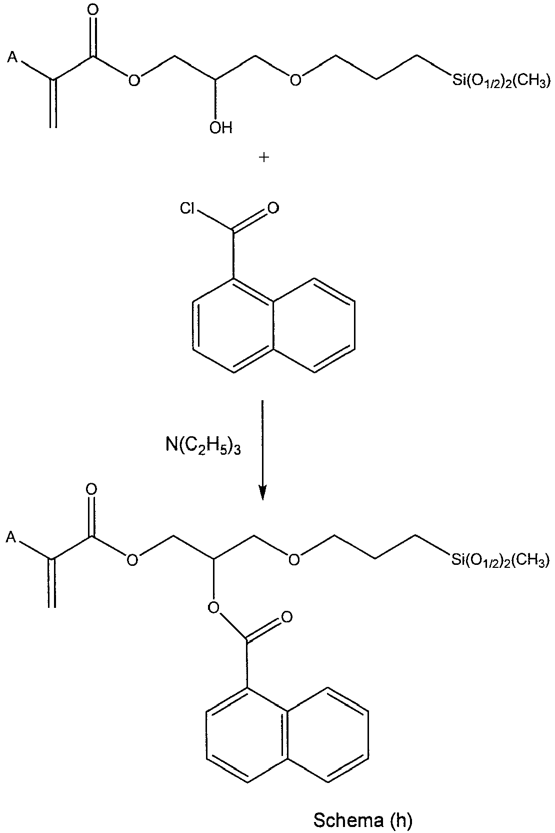

Die vorliegende Erfindung betrifft auch ein Kit, umfassend
- ein, zwei oder mehr als zwei erfindungsgemäße härtbare Dentalmaterialien (wie vorstehend definiert, vorzugsweise härtbare Dentalmaterialien wie vorstehend als bevorzugt definiert)
   und/oder
- eine, zwei oder mehr als zwei Basispasten und eine, zwei oder mehr als zwei Katalysatorpasten, wobei die eine Basispaste oder die zwei oder mehr als zwei Basispasten jeweils und unabhängig voneinander eine oder mehr als eine erfindungsgemäße Polysiloxanverbindung (wie vorstehend definiert, vorzugsweise wie vorstehend als bevorzugt definiert) umfasst bzw. umfassen.

Das zu den erfindungsgemäßen Polysiloxanverbindungen bzw. härtbaren Dentalmaterialien Gesagte gilt für das erfindungsgemäße Kit entsprechend.

Bevorzugt ist ein erfindungsgemäßes Kit (wie vorstehend definiert), wobei
- das eine oder die zwei oder mehr als zwei erfindungsgemäßen härtbaren Dentalmaterialien (wie vorstehend definiert, vorzugsweise härtbare Dentalmaterialien wie vorstehend als bevorzugt definiert)
   bzw.
- die eine oder die zwei oder mehr als zwei Basispasten
eine Farbe besitzt bzw. besitzen, die von der "VITA classical A1 - D4 Farbskala" umfasst ist.

Besonders bevorzugt ist ein erfindungsgemäßes Kit (wie vorstehend definiert, vorzugsweise wie vorstehend als bevorzugt beschrieben) umfassend
- zwei oder mehr als zwei erfindungsgemäße härtbare Dentalmaterialien (wie vorstehend definiert, vorzugsweise härtbare Dentalmaterialien wie vorstehend als bevorzugt definiert)
   und/oder
- zwei oder mehr als zwei Basispasten und eine, zwei oder mehr als zwei Katalysatorpasten, wobei die zwei oder mehr als zwei Basispasten jeweils und unabhängig voneinander eine oder mehr als eine erfindungsgemäße Polysiloxanverbindung (wie vorstehend definiert, vorzugsweise wie vorstehend als bevorzugt definiert) umfasst bzw. umfassen, wobei
- die zwei oder mehr als zwei erfindungsgemäßen härtbaren Dentalmaterialien (wie vorstehend definiert, vorzugsweise härtbare Dentalmaterialien wie vorstehend als bevorzugt definiert) bzw.
- die zwei oder mehr als zwei Basispasten
jeweils eine Farbe besitzen, die von der "VITA classical A1 - D4 Farbskala" umfasst ist, wobei die jeweiligen Farben vorzugsweise unterschiedlich sind.

Vorzugsweise umfasst das erfindungsgemäße Kit (wie vorstehend definiert, vorzugsweise wie vorsehend als bevorzugt beschrieben) zusätzlich einen, mehr als einen oder sämtliche der Gegenstände ausgewählt aus der Gruppe bestehend aus:
- ein, zwei oder mehr als zwei Bondings,
- ein, zwei oder mehr als zwei Ätzgele,
- eine oder mehr als eine Farbskala,
- einen oder mehr als einen Pinsel,
- ein oder mehr als ein Material mit einer Viskosität, die von der/den Viskosität(en) des/der erfindungsgemäßen härtbaren Dentalmaterials bzw. Dentalmaterialien und/oder von der/den Viskosität(en) der Basispaste(n) bzw. der Katalysatorpaste(n) verschieden ist, vorzugsweise ein oder mehr als ein Flow-material.

Ferner betrifft die vorliegende Erfindung ein Verfahren zur Herstellung eines erfindungsgemäßen härtbaren Dentalmaterials (ein härtbares Dentalmaterial wie vorstehend definiert, vorzugsweise ein härtbares Dentalmaterial wie vorstehend als bevorzugt definiert) mit den Schritten:
- Bereitstellen oder Herstellen einer oder mehrerer erfindungsgemäßer Polysiloxanverbindungen (Polysiloxanverbindungen wie vorstehend definiert, vorzugsweise wie vorstehend als bevorzugt definiert),
- Bereitstellen oder Herstellen einer, mehrerer oder sämtlicher Substanzen wie vorstehend als Substanzen definiert, vorzugsweise wie vorstehend als bevorzugte Substanzen definiert,
- Herstellen eines einkomponentigen oder mehrkomponentigen Systems umfassend die bereitgestellten bzw. hergestellten erfindungsgemäße(n) Polysiloxanverbindung(en) sowie die bereitgestellten bzw. hergestellten Substanz(en) sowie optional, zusätzliche Substanzen,
wobei vorzugsweise in mehrkomponentigen Systemen die eine Polymerisation auslösenden Substanzen so auf separate Komponenten verteilt sind, dass eine Polymerisation der Polysiloxanverbindungen durch Vermischen der besagten Komponenten ausgelöst wird.

In erfindungsgemäßen Verfahren zur Herstellung härtbarer Dentalmaterialien, in denen ein härtbares Dentalmaterial hergestellt wird, das mittels Lichthärtung härtbar ist, ist die Herstellung eines einkomponentigen Systems bevorzugt. In diesem Fall enthält das härtbare Dentalmaterial in der einzelnen Komponente insbesondere sämtliche für die Lichthärtung erforderlichen Bestandteile und eine Polymerisation wird durch Bestrahlen mit Licht einer definierten Wellenlänge initiiert (zur Lichthärtung härtbarer Dentalmaterialien siehe weiter oben im Text). Vorzugsweise wird das einkomponentige System als Bestandteil eines erfindungsgemäßen Kits (wie oben im Text definiert) eingesetzt.

In erfindungsgemäßen Verfahren zur Herstellung härtbarer Dentalmaterialien, in denen ein härtbares Dentalmaterial hergestellt wird, das (a) mittels Lichthärtung und mittels chemischer Härtung härtbar ist (dualhärtend) oder (b) nur durch chemische Härtung härtbar ist, ist die Herstellung eines mehrkomponentigen Systems bevorzugt. Härtbare Dentalmaterialien, die mittels chemischer Härtung härtbar sind, werden vorzugsweise als zweikomponentiges System hergestellt, so dass die eine Polymerisation auslösenden Substanzen (z.B. Polymerisationsinitiatoren, Katalysatoren für die chemische Härtung oder Bestandteile von Katalysatoren für die chemische Härtung, usw.) und die polymerisierbaren Verbindungen in separaten Komponenten vorliegen und erst durch Vermischen der separaten Komponenten eine Polymerisation erfolgt. Dualhärtende Dentalmaterialien werden vorzugsweise ebenfalls als zweikomponentige Systeme hergestellt. Dabei werden vorzugsweise die Substanzen, die eine Polymerisation durch chemische Härtung auslösen, in mindestens einer separaten Komponente des mehrkomponentigen Systems vorgelegt. Vorzugsweise wird das mehrkomponentige System (vorzugsweise das zweikomponentige System) als Bestandteil eines erfindungsgemäßen Kits eingesetzt (wie oben im Text definiert). In diesem Fall befinden sich vorzugsweise die polymerisierbaren Verbindungen, vorzugsweise die erfindungsgemäßen polymerisierbaren Verbindungen in der oder den Basispaste(n); die Substanzen, die eine chemische Härtung auslösen, in der oder den Katalysatorpaste(n).

Die vorliegende Erfindung betrifft auch ein Verfahren zur Herstellung eines gehärteten Dentalmaterials (ein gehärtetes Dentalmaterial wie vorstehend definiert, vorzugsweise ein gehärtetes Dentalmaterial wie vorstehend als bevorzugt definiert), mit folgenden Schritten:
- Bereitstellen oder Herstellen eines erfindungsgemäßen härtbaren Dentalmaterials (wie vorstehend definiert, vorzugsweise ein härtbares Dentalmaterial wie vorstehend als bevorzugt definiert),
- Polymerisation der im erfindungsgemäßen Dentalmaterial enthaltenen Polysiloxanverbindung sowie gegebenenfalls weiterer im Dentalmaterial enthaltener polymerisierbarer Bestandteile.

Die Polymerisation bzw. Vernetzung der Polysiloxanverbindung (vorzugsweise einer erfindungsgemäßen Polysiloxanverbindung wie oben definiert, vorzugsweise wie oben als bevorzugt bezeichnet) erfolgt mittels der in den (Meth)acrylatgruppen enthaltenen organisch polymerisierbaren Doppelbindungen der entsprechenden Polysiloxanverbindung. Vorzugsweise wird in einem erfindungsgemäßen Verfahren zur Herstellung eines gehärteten Dentalmaterials die Polymerisation der im erfindungsgemäßen Dentalmaterial enthaltenen Polysiloxanverbindung sowie gegebenenfalls weiterer im Dentalmaterial enthaltener polymerisierbarer Bestandteile so durchgeführt, dass ein für eine Weiterverarbeitung geeignetes Werkstück erhalten wird, vorzugsweise ein Fräsblock (Fräsrohling) oder ein Blank. Besonders bevorzugt werden schneidfähige Fräsrohlinge erhalten. Schneidfähige Fräsrohlinge eigenen sich insbesondere zur Herstellung passgenauer dentaler Bauteile auf dem Gebiet der Medizin und insbesondere auf dem Gebiet der Zahnmedizin. Bevorzugte dentale Bauteile sind Ersatzmaterialien für Zähne und Knochenstrukturen, Besonders bevorzugte dentale Bauteile sind ausgewählt aus der Gruppe bestehend aus Restaurationen, Ersatzteile, Inlays, Onlays, Verblendungen, Vollkronen, Teilkronen, Brücken, Implantate, künstliche Zähne, Zahnschienen, Zahnspangen, Zahnklammern und Stifte. Die für eine Weiterverarbeitung geeigneten Werkstücke, vorzugsweise die Fräsblöcke (Fräsrohlinge), Blanks und schneidfähigen Fräsrohlinge, liegen vorzugsweise in jeder gewünschten Form und/oder Größe vor, besonders bevorzugt in der Form eines Zylinders, Barrens, Würfels, Polyeders, Ovoids oder als Platte. Die dreidimensionale Form der vorstehend genannten und für eine Weiterverarbeitung geeigneten Werkstücke wird vorzugsweise so gewählt, dass für eine Weiterverarbeitung geeignete Werkstücke erhalten werden, die der Form/Gestalt der daraus herzustellenden dentalen Bauteile angenähert oder angepasst sind.

Es ist bevorzugt, dass in einem zusätzlichen Schritt das erfindungsgemäße gehärtete Dentalmaterial, das vorzugsweise als geeignetes Werkstück, besonders bevorzugt als Fräsblock oder Blank vorliegt, in einem zusätzlichen Schritten weiterverarbeitet wird. Bevorzugt ist ein erfindungsgemäßes Verfahren zur Herstellung eines gehärteten Dentalmaterials (wie vorstehend definiert) mit dem zusätzlichen Schritt:
- Verarbeiten des durch Polymerisation der im erfindungsgemäßen Dentalmaterial enthaltenen Polysiloxanverbindung sowie gegebenenfalls weiterer im Dentalmaterial enthaltener polymerisierbarer Bestandteile erhaltenen gehärteten Dentalmaterials, vorzugsweise des Fräsblocks oder des Blanks, so dass ein dentales Bauteil oder eine Vorstufe eines dentalen Bauteils erhalten wird, wobei das Verarbeiten vorzugsweise ein spannendes Bearbeitungsverfahren ist, wobei das spanende Bearbeitungsverfahren vorzugsweise eines oder mehrere Verfahren umfasst, ausgewählt aus der Gruppe bestehend aus Drehen, Fräsen, Bohren, Sägen, Feilen, Schneiden und Schleifen.

Besonders bevorzugt ist ein erfindungsgemäßes Verfahren zur Herstellung eines gehärteten Dentalmaterials (wie vorstehend definiert, vorzugsweise wie vorstehend als bevorzugt bezeichnet), wobei das Verarbeiten, vorzugsweise das spanende Bearbeitungsverfahren (vorzugsweise die als besonders bevorzugt bezeichneten spanenden Bearbeitungsverfahren) eine computergestützte Formgebung (computer-aided design, CAD) und/oder eine computergestützte Fertigung (computer-aided manufacturing, CAM) umfasst. Nach Durchführung des Verarbeitens liegt das gehärtete Dentalmaterial geformt als dentales Bauteil oder als eine Vorstufe eines dentalen Bauteils vor. Bevorzugte dentale Bauteile sind ausgewählt aus der Gruppe bestehend aus Restaurationen, Ersatzteile, Inlays, Onlays, Verblendungen, Vollkronen, Teilkronen, Brücken, Implantate, künstliche Zähne, Zahnschienen, Zahnspangen, Zahnklammern und Stifte.

Es hat sich gezeigt, dass die erfindungsgemäßen, gehärteten Dentalmaterialien in manchen Anwendungen besonders gut für CAD/CAM-unterstützte spanende Bearbeitungsverfahren geeignet sind. Die vorliegende Erfindung betrifft daher auch die Verwendung eines erfindungsgemäßen gehärteten Dentalmaterials (wie vorstehend definiert, vorzugsweise ein gehärtetes Dentalmaterial wie vorstehend als bevorzugt beschrieben, vorzugsweise ein Fräsblock bzw. Blank) zur Herstellung dentaler Bauteile oder Vorstufen dentaler Bauteile, vorzugsweise zur Herstellung dentaler Bauteile oder Vorstufen dentaler Bauteile mittels computergestützter Formgebung (computer-aided design, CAD) und/oder computergestützter Fertigung (computer-aided manufacturing, CAM).

Die vorliegende Erfindung steht zudem im Zusammenhang mit einem dentalen Therapieverfahren mit folgendem Schritt:

Applizieren eines erfindungsgemäßen härtbaren Dentalmaterials (wie vorstehend definiert, vorzugsweise ein härtbares Dentalmaterial wie vorstehend als bevorzugt definiert)
- auf der Zahnsubstanz oder dem dentalen Gewebe eines Patienten
   oder
- auf einer Dentalrestauration.

Die folgenden Beispiele dienen zur Verdeutlichung der Erfindung.

### A) Herstellung und Eigenschaften von Polysiloxanverbindungen:

### 1.) Beispielsynthese einer intermediären Polysiloxanverbindung mit reaktiver polarer Gruppe (vgl. EP 1 685 182 B1) (Polysiloxan A)

### 1.1) Synthese einer monomeren Silaneinheit (vgl. EP 1 685 182 B1, Bsp. 3):

Zur Vorlage von 100 g (0,402 mol) 3-Glycidyloxypropyldiethoxymethylsilan werden unter trockener Atmosphäre (Sauerstoff) ein Additionskatalysator, BHT als Stabilisator und anschließend 38,05 g (0,442 mol) Methacrylsäure zugetropft und bei ca. 80°C gerührt (ca. 24 h) (vgl. Schema (c) oben, bzw. Absatz [0048] in EP 1 685 182 B1). Die Umsetzung wird über die Abnahme der Carbonsäurekonzentration mittels Säuretitration sowie dem Epoxidumsatz mittels Ramanspektroskopie/Epoxidtitration verfolgt. Die für die Epoxidgruppe charakteristische Bande wird im Ramanspektrum bei 1256 cm⁻¹ detektiert. Der Epoxid- bzw. Carbonsäureumsatz liegt bei ≥ 99 % bzw. ≥ 88 % (Folge des Carbonsäureüberschusses).

### 1.2) Hydrolyse bzw. Kondensation der monomeren Silaneinheit zu einer intermediären Polysiloxanverbindung (Polysiloxan A) (vgl. Schema (d) oben, bzw. EP 1 685 182 B1, Bsp. 6):

Nach Zugabe von Essigester (1000 ml/mol monomerer Silaneinheit) und H₂O zur Hydrolyse mit HCl als Katalysator zur synthetisierten monomeren Silaneinheit wird bei 30°C gerührt. Der Verlauf der Hydrolyse wird durch Wassertitration verfolgt. Die Aufarbeitung erfolgt nach mehrtägigem Rühren bei 30°C durch mehrmaliges Ausschütteln mit wässriger NaOH und anschließendem Ausschütteln mit Wasser und Filtration über einen hydrophobierten Filter. Nach Zusatz von BHT wird zunächst bei 40 °C abrotiert und anschließend werden Lösungsmittelreste (z.B. Wasser- und Alkoholreste) unter Vakuum mittels einer Ölpumpe abgezogen, um die Alkohol- und Wasserreste zu entfernen. Es resultiert ein flüssiges Harz mit einer Viskosität von 4,5 Pa*s bei 25 °C (stark abhängig von den genauen Hydrolyse- und Aufarbeitungsbedingungen).

Die als Polysiloxan A bezeichnete intermediäre Polysiloxanverbindung dient nachfolgend einerseits als Referenzverbindung bei einem Vergleich physikalischer Eigenschaften (vgl. Tabelle 2 weiter unten im Text) und andererseits als Ausgangsverbindung zur weiteren Umsetzung zu einer erfindungsgemäßen Polysiloxanverbindung.

### 2) Herstellung erfindungsgemäßer Polysiloxanverbindungen (Polysiloxane B, C, D, E und F):

### 2.1) Herstellung einer erfindungsgemäßen Polysiloxanverbindung (Polysiloxan B) durch Umsetzen von Polysiloxan A mit Naphtoylchlorid :

Zu einer Vorlage von 15 mmol Polysiloxan A und 1.1 eq Triethylamin in Toluol wird unter trockener Atmosphäre unter Eiskühlung (4°C) eine Lösung von 1.1 eq. Naphtoylchlorid in Toluol langsam hinzugetropft. Nach vollständiger Zugabe wird die Lösung bei Raumtemperatur gerührt. Die Umsetzung wird über die Abnahme der für die OH-Gruppe charakteristischen Bande mittels IR-Spektroskopie verfolgt; die für die OH-Gruppe charakteristische Bande wird im IR bei 3200-3400 cm⁻¹ detektiert. Zur Aufarbeitung wird die Lösung zunächst mit 1M HCl ausgeschüttelt und anschließend zwei Mal mit Wasser gewaschen. Nach Trocknung der organischen Phase über Magnesiumsulfat und Entfernen des Lösungsmittels unter Vakuum wird ein leicht gelbliches fließfähiges Harz mit einer Viskosität von 17,5 Pa*s bei 25 °C erhalten. Ein Vergleich physikalischer Eigenschaften ist Tabelle 2 zu entnehmen.

### 2.2) Herstellung einer erfindungsgemäßen Polysiloxanverbindung (Polysiloxan C) durch Umsetzen von Polysiloxan A mit tert-Butylbenzoylchlorid:

Zu einer Vorlage von 15 mmol Polysiloxan A und 1.1 eq Triethylamin in Toluol werden unter trockener Atmosphäre unter Eiskühlung (4°C) eine Lösung von 1.1 eq. tert-Butylbenzoylchlorid in Toluol langsam hinzugetropft. Nach vollständiger Zugabe wird die Lösung bei Raumtemperatur gerührt. Die Umsetzung wird über die Abnahme der für die OH-Gruppe charakteristischen Bande mittels IR-Spektroskopie verfolgt; die für die OH-Gruppe charakteristische Bande wird im IR bei 3200-3400 cm⁻¹ detektiert. Zur Aufarbeitung wird die Lösung zunächst mit 1M HCl ausgeschüttelt und anschließend zwei Mal mit Wasser gewaschen. Nach Trocknung der organischen Phase über Magnesiumsulfat und Entfernen des Lösungsmittels unter Vakuum wird ein leicht gelbliches fließfähiges Harz mit einer Viskosität von 13,5 Pa*s bei 25 °C erhalten. Ein Vergleich physikalischer Eigenschaften ist Tabelle 2 zu entnehmen.

### 2.3) Herstellung einer erfindungsgemäßen Polysiloxanverbindung (Polysiloxan D) durch Umsetzen von Polysiloxan A mit n-Dodecanoylchlorid:

Zu einer Vorlage von 15 mmol Polysiloxan A und 1,1 eq. Triethylamin in Toluol werden unter trockener Atmosphäre unter Eiskühlung (4°C) eine Lösung von 1.1 eq. n-Dodecanoylchlorid in Toluol langsam hinzugetropft. Nach vollständiger Zugabe wird die Lösung bei Raumtemperatur gerührt. Die Umsetzung wird über die Abnahme der für die OH-Gruppe charakteristischen Bande mittels IR-Spektroskopie verfolgt; die für die OH-Gruppe charakteristische Bande wird im IR bei 3200-3400 cm⁻¹ detektiert. Zur Aufarbeitung wird die Lösung zunächst mit 1 M HCl ausgeschüttelt und anschließend zwei Mal mit Wasser gewaschen. Nach Trocknung der organischen Phase über Magensiumsulfat und Entfernen des Lösungsmittels unter Vakuum wird ein leicht gelbliches fließfähiges Harz mit einer Viskosität von 1,5 Pa*s bei 25 °C erhalten. Ein Vergleich physikalischer Eigenschaften ist Tabelle 2 zu entnehmen.

### 2.4) Herstellung einer erfindungsgemäßen Polysiloxanverbindung (Polysiloxan E) durch Umsetzen von Polysiloxan A mit (i) para-Toluolsulfonylchlorid und anschließend mit (ii) 1-Naphthalencarboxamid (vgl. Schema (m)):

Es wird eine Lösung aus 16,5 mmol (1,1eq.) *para*-Toluolsulfonylchlorid in 50 ml einer Mischung aus Aceton und Toluol (Volumenverhältnis: 1:1) unter Eiskühlung (4°C) mit 16,5 mmol (1,1 eq.) Triethylamin vermischt. Die resultierende Mischung wird anschließend mit 15 mmol (1,0 eq) Polysiloxan A versetzt. Die nach abgeschlossener Zugabe von Polysiloxan A resultierende klare Lösung wird 1 h bei 4°C gerührt. Anschließend wird ein Gemisch aus 1,1 eq. 1-Naphthalencarboxamid und 1,1 eq. Triethylamin in 5 ml Aceton / Toluol (Volumenverhältnis: 1:1) hinzugegeben. Die resultierende Mischung wird für die Dauer von 3 Stunden auf 75°C erhitzt.

In einem nächsten Schritt wird die Lösung dreimal mit Wasser gewaschen und die organische Phase über Magnesiumsulfat getrocknet. Nach Abzug des Lösungsmittels im Vakuum resultiert ein flüssiges, leicht gelbliches Harz mit einer Viskosität von ca. 24 Pa*s bei 25°C, einer Brechzahl n_{D} von ca. 1,52 und einer Schrumpfung von 3,6 % bei Aushärtung. Ein Vergleich physikalischer Eigenschaften ist Tabelle 2 zu entnehmen.

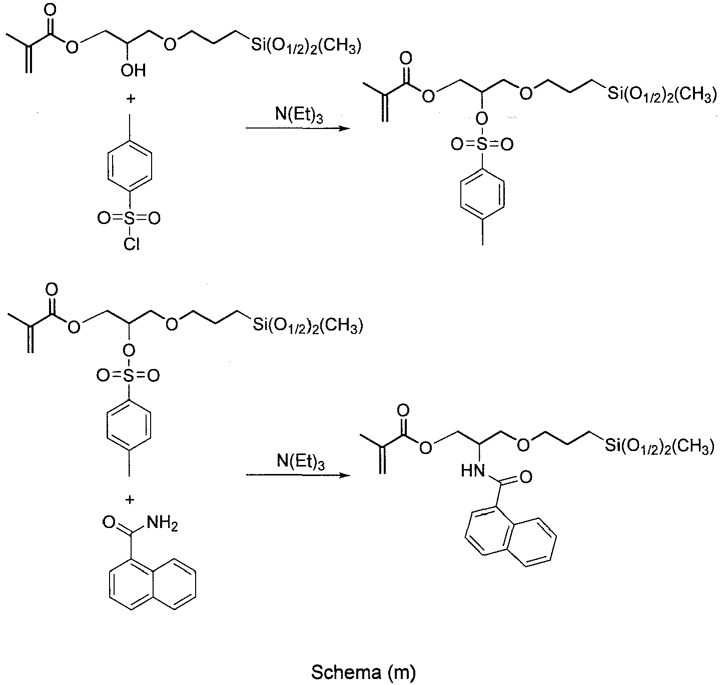

### 2.5) Herstellung einer erfindungsgemäßen Polysiloxanverbindung (Polysiloxan F) durch Umsetzen von Polysiloxan A mit (i) para-Toluolsulfonylchlorid und anschließend mit (ii) 1-Naphthalenthiocarbonsäure (vgl. Schema (n)):

Es wird eine Lösung aus 16,5 mmol (1,1eq.) *para*-Toluolsulfonylchlorid in 50 ml einer Mischung aus Aceton und Toluol (Volumenverhältnis: 1:1) unter Eiskülung (4°C) mit 16,5 mmol (1,1 eq.) Triethylamin vermischt. Die resultierende Mischung wird anschließend mit 15 mmol (1,0 eq) Polysiloxan A versetzt. Die nach abgeschlossener Zugabe von Polysiloxan A resultierende klare Lösung wird 1 h bei 4°C gerührt. Anschließend wird ein Gemisch aus 1,1 eq. 1-Naphthalenthiocarbonsäure und 1,1 eq. Triethylamin in 5 ml Aceton / Toluol (Volumenverhältnis: 1:1) hinzugegeben. Die resultierende Mischung wird für die Dauer von 3 Stunden auf 75°C erhitzt.

In einem nächsten Schritt wird die Lösung dreimal mit Wasser gewaschen und die organische Phase über Magnesiumsulfat getrocknet. Nach Abzug des Lösungsmittels im Vakuum resultiert ein flüssiges, leicht gelbliches Harz mit einer Viskosität von ca. 15 Pa*s bei 25°C, einer Brechzahl n_{D} von ca. 1,53 und einer Schrumpfung von 3,6 % bei Aushärtung. Ein Vergleich physikalischer Eigenschaften ist Tabelle 2 zu entnehmen.

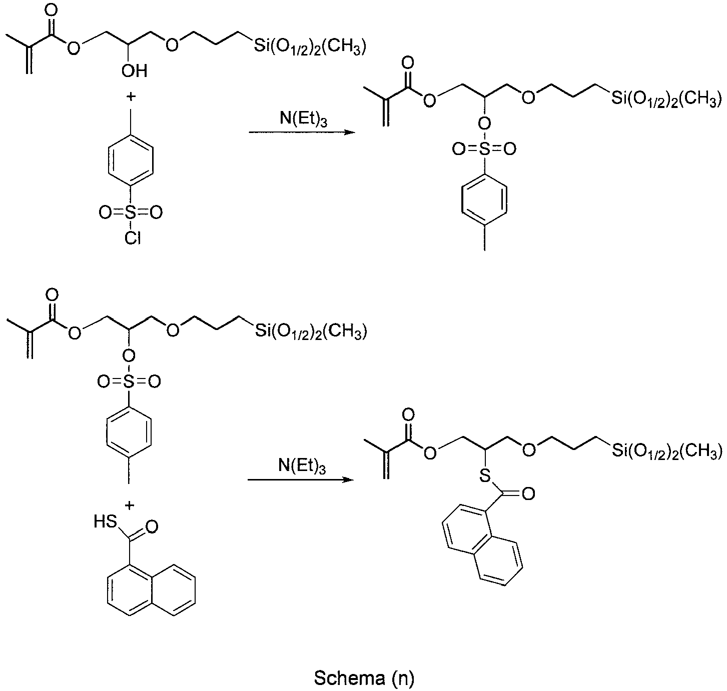

**Tabelle 2: Physikalische Eigenschaften erfindungsgemäßer Polysiloxanverbindungen (B, C, D, E und F) im Vergleich mit dem nicht erfindungsgemäßen Polysiloxan A. *Die Eigenschaften "Biegefestigkeit" und "Schrumpfung" wurden im ausgehärteten Zustand bestimmt (vgl. hierzu nachfolgend die Punkte C-3 und C-4).**

| | Polysiloxan A (Referenz) | Polysiloxan B | Polysiloxan C | Polysiloxan D | Polysiloxan E | Polysiloxan F |
|---|---|---|---|---|---|---|
| Biegefestigkeit* | 32 | 43 | 39 | 35 | 44 | 42 |
| Schrumpfung* | 4,8 | 3,6 | 3,4 | 3,7 | 3,6 | 3,6 |
| Viskosität | 4,5 | 17,5 | 13,5 | 1,5 | 24 | 15 |
| Brechungsindex | 1,47 | 1,52 | 1,49 | 1.48 | 1,52 | 1,53 |

Die Viskosität erfindungsgemäßer Polysiloxane (Polysiloxan B, C, D, E und F) ist gegenüber der Viskosität nicht erfindungsgemäßer Polysiloxane (Polysiloxan A) nicht nachteilig verändert (allenfalls im Falle von Polysiloxan B, C, E und F unwesentlich erhöht).

Erfindungsgemäße Polysiloxanverbindungen besitzen einen Brechungsindex in dem bevorzugten Bereich von 1,48 bis 1,54 und damit einen höheren Brechungsindex, verglichen mit dem Brechungsindex von 1,47 des nicht erfindungsgemäßen Polysiloxans A.

### B) Herstellung und Eigenschaften härtbarer Dentalmaterialien (Komposite A bis G):

In einem 100 ml Laborkneter werden die jeweiligen Polysiloxanverbindungen (Polysiloxan A, B, C, D, E und F) sowie zusätzliche Verbindungen/Substanzen gemäß Tabelle 3 in den dort angegebenen Mengenverhältnissen miteinander vermengt, so dass homogene, pastöse Formulierungen resultieren (Vorstufe eines nicht-erfindungsgemäßen Komposits A sowie Vorstufen erfindungsgemäßer Komposite B bis G). Nach Entlüften der pastösen Formulierungen unter Vakuum (bei einem Druck von 0,9 bar) werden die entlüfteten, pastösen Formulierungen (Komposite A bis G) hinsichtlich ihrer Biegefestigkeit und ihres Polymerisationsschrumpfes ("Schrumpfung") charakterisiert. Die Ergebnisse sind in Tabelle 4 vergleichend gegenübergestellt.

Alle Angaben in Tabelle 3 sind Gewichtsprozentangaben, bezogen auf das Gesamtgewicht des jeweiligen Komposits.

**Tabelle 3: Zusammensetzungen härtbarer Dentalmaterialen auf Basis erfindungsgemäßer Polysiloxanverbindungen (Polysiloxan B, C, D, E und F (eingesetzt in den Kompositen B bis G)) bzw. auf Basis des nicht-erfindungsgemäßen Polysiloxans A (eingesetzt in Komposit A). Alle härtbaren Dentalmaterialien (Komposite) enthalten als Initiatoren / Inhibitoren gleiche Mengen an Campherchinon (0,4 Gew.-%) und Dimethylaminoparabenzoesäure (0,6 Gew.-%) zur Initiierung sowie gleiche Mengen an Butylhydroxytoluol (0,1 Gew.-%) zur Inhibierung.**

| Bestandteile | Komposit A (Referenz) | Komposit B | Komposit C | Komposit D | Komposit E | Komposit F | Komposit G |
|---|---|---|---|---|---|---|---|
| Polysiloxan A | 20 | 0 | 0 | 0 | 0 | 0 | 0 |
| Polysiloxan B | 0 | 20 | 0 | 0 | 10 | 0 | 0 |
| Polysiloxan C | 0 | 0 | 20 | 0 | 0 | 0 | 0 |
| Polysiloxan D | 0 | 0 | 0 | 20 | 0 | 0 | 0 |
| Polysiloxan E | 0 | 0 | 0 | 0 | 0 | 20 | 0 |
| Polysiloxan F | 0 | 0 | 0 | 0 | 0 | 0 | 20 |
| BisGMA / TEGDMA im Massenverhält nis 1:1 | 0 | 0 | 0 | 0 | 10 | 0 | 0 |
| Bariumsilik at-Glas (0,7 µm) silanisiert | 15,8 | 15,8 | 15,8 | 15,8 | 15,8 | 15,8 | 15,8 |
| Bariumsilik at-Glas (1,5 µm) silanisiert | 63.1 | 63,1 | 63,1 | 63,1 | 63,1 | 63,1 | 63,1 |
| Initiatoren / Inhibitoren | 1,1 | 1,1 | 1,1 | 1,1 | 1,1 | 1,1 | 1,1 |

**Tabelle 4: Physikalische Eigenschaften und die dazugehörigen Werte der Komposite A, B, C, D, E, F und G. Die Eigenschaften "Biegefestigkeit" und "Schrumpfung" wurden im ausgehärteten Zustand bestimmt (vgl. hierzu nachfolgend die Punkte C-3 und C-4).**

| | Komposit A | Komposit B | Komposit C | Komposit D | Komposit E | Komposit F | Komposit G |
|---|---|---|---|---|---|---|---|
| Biegefestigkeit | 110 | 125 | 127 | 120 | 155 | 126 | 128 |
| Schrumpfung | 1,5 | 1,2 | 1,1 | 1,3 | 1,9 | 1,2 | 1,2 |

Die erfindungsgemäßen härtbaren Dentalmaterialien (Komposite B bis G) besitzen im ausgehärteten Zustand eine höhere Biegefestigkeit verglichen mit der Biegefestigkeit eines nicht erfindungsgemäßen härtbaren Dentalmaterials (Komposit A) umfassend ein nicht erfindungsgemäßes Polysiloxan A (wie oben beschrieben).

Erfindungsgemäße härtbare Dentalmaterialien (Komposite B bis D sowie F und G) sind außerdem dadurch gekennzeichnet, dass sie eine geringere Schrumpfung beim Aushärten erfahren, verglichen mit einem nicht erfindungsgemäßen härtbaren Dentalmaterial (Komposit A) umfassend ein nicht erfindungsgemäßes Polysiloxan A (wie oben beschrieben).

### C) Bestimmungsmethoden zur Bestimmung der physikalischen Eigenschaften der Polysiloxanverbindungen bzw. der härtbaren Dentalmaterialien (Komposite):

### C-1) Bestimmung der Viskosität:

Die Bestimmung der Viskosität erfolgt mittels eines Rheometers (Physica MCR 301) der Firma Anton Paar. Hierfür werden jeweils 4g des nicht polymerisierten Polysiloxans (Polysiloxan A, B, C, D, E bzw. F) auf einer 50 mm Rheometherplatte gleichmäßig verteilt. Anschließend erfolgt die Scherung des Materials bei 25°C mittels rotierender Bewegung (die Schergeschwindigkeit (Scherrate) ý wurde so gewählt, dass sie einen Bereich von 0,5 bis 10 s⁻¹ durchläuft). Die Viskosität des Materials wurde an 20 Messpunkten ermittelt die jeweils in einem 10s-Abstand aufgenommen wurden. Der letzte Messpunkt spiegelt die Viskosität bei maximaler Scherung wieder. Die Viskosität ist in der obigen Tabelle 2 in Pa*s angegeben.

### C-2) Bestimmung des Brechungsindexes:

Die Bestimmung des Brechungsindexes erfolgt mittels eines Refraktometers (RE40) der Firma Mettler Toledo an jeweils 1g des nicht polymerisierten Polysiloxans (Polysiloxan A, B, C, D, E bzw. F). Der Brechungsindex ist eine dimensionslose physikalische Größe.

### C-3) Bestimmung der Biegefestigkeit:

Die Bestimmung der Biegefestigkeit der Polysiloxane (Polysiloxan A, B, C, D, E bzw. F, vgl. Tabelle 2, "Biegefestigkeit") bzw. der härtbaren Dentalmaterialien (Komposite A bis G, vgl. Tabelle 4, "Biegefestigkeit") erfolgte in Anlehnung an FprEN ISO 4049 an jeweils 5 entsprechend ausgehärteten Materialien (Probekörpern) (vgl. hierzu FprEN ISO 4049, Punkte 7.11.3 und 7.11.4). Die Biegefestigkeit ist in den vorstehenden Tabellen 2 und 4 in Megapascal (MPa) angegeben.

Für die Untersuchungen an den jeweiligen Polysiloxanen A bis F wurden diese durch Zugabe von jeweils 3g/kg Campherchinon und 4.5 g/kg Dimethylaminoparabenzoesäure für die Härtung aktiviert. Die so erhaltenen aktivierten Polysiloxane wurden anschließend jeweils in eine Teflonform (2 mm * 2 mm * 50 mm) überführt und die Oberseite der Teflonform mit einer Ethylacetatfolie abgedeckt. Anschließend wurden die so erhaltenen Proben mit Licht einer Wellenlänge im Bereich von 400 bis 500 nm in einem Lichtkasten für einen Zeitraum von 60 Minuten belichtet, so dass ausgehärtete Probekörper erhalten wurden. Diese ausgehärteten Probekörper wurden anschließend aus der Teflonform gelöst und für die Dauer von 24 Stunden in einem Wasserbad mit einer Wassertemperatur von 37°C gelagert. Die Messung der Biegefestigkeit wurde danach mittels eines Kraftmessgerätes der Firma Zwick Roell Z005 KAS-TC (Baujahr 2007) bei einer Kraftzunahme von 50 ± 16 N/min durchgeführt.

Die Untersuchungen an den jeweiligen Kompositen (Komposite A bis G, vgl. die jeweiligen Zusammensetzungen gemäß Tabelle 3) wurden unter gleichen Bedingungen und mit den gleichen Methoden (aber ohne erneutem Zusatz von Initiatoren wie Campherchinon und Dimethylaminoparabenzoesäure) durchgeführt, wie sie vorstehend für die Untersuchungen an den Polysiloxanen angegeben sind.

### C-4) Bestimmung des Polymerisationsschrumpfes:

Die Bestimmung des Polymerisationsschrumpfes der Polysiloxane (Polysiloxan A, B, C, D, E bzw. F, vgl. Tabelle 2, "Schrumpfung") bzw. der härtbaren Dentalmaterialien (vgl. Tabelle 4, "Schrumpfung") erfolgt nach der Methode von "Watts"* an entsprechend gehärteten Dentalmaterialien. Der Polymerisationsschrumpf ("Schrumpfung") ist in den obigen Tabellen 2 und 4 in Volumenprozent (Vol-%) angegeben, bezogen auf das Volumen des Probekörpers vor der Aushärtung. Die in den Tabellen 2 und 4 angegebenen Werte bedeuten eine Volumenverringerung in Prozent nach Aushärtung.

Für die Untersuchungen an den jeweiligen Polysiloxanen wurden diese mit 3g/kg Campherchinon und 4.5 g/kg Dimethylaminoparabenzoesäure für die Härtung aktiviert. Die Schrumpfungsuntersuchung erfolgte im Rahmen einer photoinduzierten radikalischen Polymerisation mit Licht einer Wellenlänge im Bereich von 400 bis 500 nm nach der Methode von "Watts".

Die Untersuchungen an den jeweiligen Kompositen (Komposite A bis G, vgl. die jeweiligen Zusammensetzungen gemäß Tabelle 3) wurden analog zu den Untersuchungen an den Polysiloxanen durchgeführt, aber ohne erneuten Zusatz von Initiatoren wie Campherchinon und Dimethylaminoparabenzoesäure.

*Methode nach Watts D.C., Cash A.J.: Determination of polymerization shrinkage kinetics in visible light cured materials: methods development, Dental Materials 1991; 7; 281

## Patentansprüche

1. Polysiloxanverbindung umfassend
(A) ein, zwei, drei oder mehr als drei strukturell jeweils identische erste Siloxaneinheiten ausgewählt aus der Gruppe bestehend aus Siloxaneinheiten der allgemeinen Formel (I) 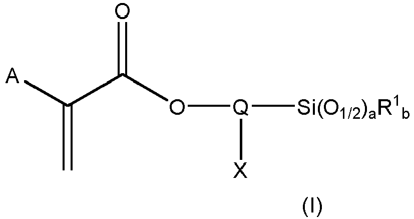
wobei für die Struktureinheiten A, Q, X und R¹ in jeder der strukturell identischen ersten Siloxaneinheiten unabhängig voneinander gilt:
A bedeutet H oder CH₃,
Q bedeutet eine den Substituenten X tragende Verknüpfungsgruppe,
R¹ bedeutet einen Alkylrest mit insgesamt 1 bis 4 Kohlenstoffatomen
und
X ist ausgewählt aus der Gruppe bestehend aus
verzweigter, gesättigter, unsubstituierter Alkylrest mit insgesamt 7 bis 18 Kohlenstoffatomen,
unverzweigter, gesättigter, unsubstituierter Alkylrest mit insgesamt 7 bis 18 Kohlenstoffatomen,
unsubstituierter oder alkylsubstituierter Arylrest mit insgesamt 10 bis 18 Kohlenstoffatomen, wobei der Alkylsubstituent im alkylsubstituierten Arylrest
verzweigt und gesättigt
oder
unverzweigt und gesättigt ist
und
Z-(CO)-R², wobei hierin für die Struktureinheiten Z und R² unabhängig voneinander und unabhängig von der Bedeutung der Struktureinheiten A, Q und R¹ gilt:
Z bedeutet O, S oder NH, vorzugsweise O und
R² ist ausgewählt aus der Gruppe bestehend aus
verzweigter, gesättigter, unsubstituierter Alkylrest mit insgesamt 6 bis 18 Kohlenstoffatomen,
unverzweigter, gesättigter, unsubstituierter Alkylrest mit insgesamt 6 bis 18 Kohlenstoffatomen,
unsubstituierter oder alkylsubstituierter Arylrest mit insgesamt 9 bis 18 Kohlenstoffatomen, wobei der Alkylsubstituent im alkylsubstituierten Arylrest
verzweigt und gesättigt
oder
unverzweigt und gesättigt ist,
wobei
b 0, 1 oder 2 bedeutet und
a 3-b bedeutet
und
wobei ein Kohlenstoffatom eines Alkylrests mit einem anderen Kohlenstoffatom dieses Alkylrests unter Bildung eines Ringes verknüpft sein kann.

2. Polysiloxanverbindung, vorzugsweise nach Anspruch 1, umfassend
(A) ein, zwei, drei oder mehr als drei strukturell jeweils identische erste Siloxaneinheiten ausgewählt aus der Gruppe bestehend aus Siloxaneinheiten der allgemeinen Formel (la) 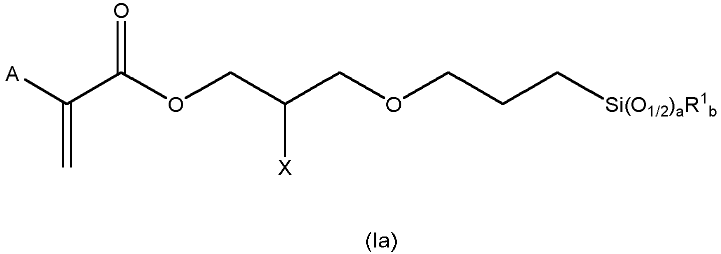 wobei für die Struktureinheiten A, X und R¹ in jeder der strukturell identischen ersten Siloxaneinheiten unabhängig voneinander gilt:
A bedeutet H oder CH₃,
R¹ bedeutet einen Alkylrest mit insgesamt 1 bis 4 Kohlenstoffatomen
und
X ist ausgewählt aus der Gruppe bestehend aus
verzweigter, gesättigter, unsubstituierter Alkylrest mit insgesamt 7 bis 18 Kohlenstoffatomen, vorzugsweise 11 bis 18 Kohlenstoffatomen, besonders bevorzugt 11 bis 14 Kohlenstoffatomen,
unverzweigter, gesättigter, unsubstituierter Alkylrest mit insgesamt 7 bis 18 Kohlenstoffatomen, vorzugsweise 11 bis 18 Kohlenstoffatomen, besonders bevorzugt 11 bis 14 Kohlenstoffatomen,
unsubstituierter oder alkylsubstituierter Arylrest mit insgesamt 10 bis 18 Kohlenstoffatomen, vorzugsweise 11 bis 18 Kohlenstoffatomen, besonders bevorzugt 11 bis 14 Kohlenstoffatomen, wobei der Alkylsubstituent im alkylsubstituierten Arylrest
verzweigt und gesättigt
oder
unverzweigt und gesättigt ist
und
Z-(CO)-R², wobei hierin für die Struktureinheiten Z und R² unabhängig voneinander und unabhängig von der Bedeutung der Struktureinheiten A und R¹ gilt:
Z bedeutet O, S oder NH, vorzugsweise O
und
R² ist ausgewählt aus der Gruppe bestehend aus
verzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
unverzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
unsubstituierter oder alkylsubstituierter Arylrest mit insgesamt 9 bis 18 Kohlenstoffatomen, vorzugsweise 10 bis 18 Kohlenstoffatomen, besonders bevorzugt 10 bis 14 Kohlenstoffatomen, wobei der Alkylsubstituent im alkylsubstituierten Arylrest
verzweigt und gesättigt
oder
unverzweigt und gesättigt ist,
wobei
b 0, 1 oder 2 bedeutet und
a 3-b bedeutet
und
(B) ein, zwei, drei oder mehr als drei zweite, strukturell jeweils identische Siloxaneinheiten, die von der ersten Siloxaneinheit strukturell verschieden sind.

3. Polysiloxanverbindung nach einem der vorangehenden Ansprüche, umfassend
(A) ein, zwei, drei oder mehr als drei strukturell jeweils identische erste Siloxaneinheiten ausgewählt aus der Gruppe bestehend aus Siloxaneinheiten der allgemeinen Formel (Ic) 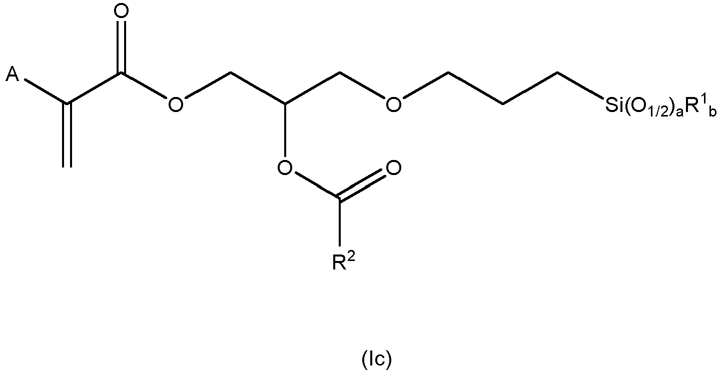 wobei für die Struktureinheiten A, R¹ und R² in jeder der strukturell identischen ersten Siloxaneinheiten unabhängig voneinander gilt:
A bedeutet H oder CH₃,
R¹ bedeutet einen Alkylrest mit insgesamt 1 bis 4 Kohlenstoffatomen,
und
R² ist ausgewählt aus der Gruppe bestehend aus
verzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
unverzweigter, gesättigter, unsubstituierter Alkylrest mit insgesamt 6 bis 18 Kohlenstoffatomen, vorzugsweise 10 bis 18 Kohlenstoffatomen, besonders bevorzugt 10 bis 14 Kohlenstoffatomen,
unsubstituierter oder alkylsubstituierter Arylrest mit insgesamt 9 bis 18 Kohlenstoffatomen, vorzugsweise 10 bis 18 Kohlenstoffatomen, besonders bevorzugt 10 bis 14 Kohlenstoffatomen, wobei der Alkylsubstituent im alkylsubstituierten Arylrest
verzweigt und gesättigt
oder
unverzweigt und gesättigt ist,
wobei
b 0, 1 oder 2 bedeutet und
a 3-b bedeutet
und
(B) ein, zwei, drei oder mehr als drei zweite, strukturell jeweils identische Siloxaneinheiten, die von der ersten Siloxaneinheit strukturell verschieden sind

4. Polysiloxanverbindung nach einem der vorangehenden Ansprüche, wobei R² ausgewählt ist aus der Gruppe bestehend aus
1-Naphthyl,
4-tert.-butyl-1-Phenyl,
und
n-Undecanyl.

5. Härtbares Dentalmaterial umfassend
- eine oder mehr als eine Polysiloxanverbindung nach einem der vorangehenden Ansprüche
und
- eine oder mehr als eine Verbindung, die keine Polysiloxanverbindung nach einem der vorangehenden Ansprüche ist.

6. Härtbares Dentalmaterial nach Anspruch 5, weiter umfassend ein, zwei, mehr als zwei oder sämtliche Substanzen aus der Gruppe bestehend aus:
- dentale organische Füllstoffpartikel, die vorzugsweise röntgenopak und/oder nanoskalig sind,
- dentale anorganische Füllstoffpartikel, die vorzugsweise röntgenopak und/oder nanoskalig sind,
- dentale organisch oberflächenmodifizierte anorganische Füllstoffpartikel, die vorzugsweise röntgenopak und/oder nanoskalig sind,
- rheologische Hilfsmittel,
- Polymerisationsinitiatoren, vorzugsweise Photoinitiatoren,
- chemische Verbindungen als Katalysatoren bzw. Bestandteile von Katalysatorsystemen,
- Farbmittel, vorzugsweise Farbpigmente,
- Stabilisatoren, insbesondere Tageslichtstabilisatoren,
- Inhibitoren,
- Aktivatoren,
- Molekulargewichtsregler,
- Konservierungsmittel,
- grenzflächenaktive Substanzen,
- Mikrobizide, vorzugsweise Bakterizide,
- organische, vorzugsweise radikalisch polymerisierbare Monomere, die keine erfindungsgemäßen Polysiloxane sind, vorzugsweise zur Umsetzung mit der erfindungsgemäßen Polysiloxanverbindung,
- organische Polymere und Oligomere und Verbindungen mit hohen Molekulargewichten, vorzugsweise Weichmacher,
- Verdickungsmittel und
- dentale Arzneimittel.

7. Gehärtetes Dentalmaterial, erhältlich aus einem härtbaren Dentalmaterial nach einem der Ansprüche 5 bis 6 mittels Polymerisation der im Dentalmaterial enthaltenen Polysiloxanverbindung sowie gegebenenfalls weiterer im Dentalmaterial enthaltener polymerisierbarer Bestandteile.

8. Verwendung eines gehärteten Dentalmaterials nach Anspruch 7 zur Herstellung dentaler Bauteile oder Vorstufen dentaler Bauteile, vorzugsweise zur Herstellung dentaler Bauteile oder Vorstufen dentaler Bauteile mittels computergestützter Formgebung (computer-aided design, CAD) und/oder computergestützter Fertigung (computer-aided manufacturing, CAM).

9. Polysiloxanverbindung nach einem der Ansprüche 1 bis 4 oder härtbares Dentalmaterial nach einem der Ansprüche 5 bis 6 oder gehärtetes Dentalmaterial nach Anspruch 7, zur Anwendung in einem therapeutischen Verfahren, wobei eine Polysiloxanverbindung nach einem der Ansprüche 1 bis 4 vorzugsweise als polymerisierbarer Bestandteil eines härtbaren Dentalmaterials eingesetzt wird.

10. Polysiloxanverbindung nach einem der Ansprüche 1 bis 4 oder härtbares Dentalmaterial nach einem der Ansprüche 5 bis 6 oder gehärtetes Dentalmaterial nach Anspruch 7 zur Anwendung nach Anspruch 9, zur spezifischen Anwendung
in einem therapeutischen Verfahren zum temporären oder permanenten Füllen einer dentalen Kavität
oder
in einem therapeutischen Verfahren als
dentales Füllungsmaterial,
dentales Unterfüllungsmaterial,
dentales Adhäsiv (Bonding),
als fliessfähiges Kompositmaterial (Flow-Material),
als Fissurenversiegler,
als Kronenmaterial,
als Inlay und/oder Onlay,
als Brückenmaterial
und/oder als Stumpfaufbaumaterial.

11. Kit, umfassend
- ein, zwei oder mehr als zwei härtbare Dentalmaterialien nach einem der Ansprüche 5 bis 6
und/oder
- eine, zwei oder mehr als zwei Basispasten und eine, zwei oder mehr als zwei Katalysatorpasten, wobei die eine Basispaste oder die zwei oder mehr als zwei Basispasten jeweils und unabhängig voneinander eine oder mehr als eine Polysiloxanverbindung nach einem der Ansprüche 1 bis 4 umfasst bzw. umfassen.

12. Verfahren zur Herstellung einer Polysiloxanverbindung nach einem der Ansprüche 1 bis 4, mit den folgenden Schritten:
- Herstellen oder Bereitstellen einer intermediären Polysiloxanverbindung umfassend
- (A_{P}) ein, zwei, drei oder mehr als drei strukturell jeweils identische erste intermediäre Siloxaneinheiten ausgewählt aus der Gruppe bestehend aus Siloxaneinheiten der allgemeinen Formel (I_{P}) 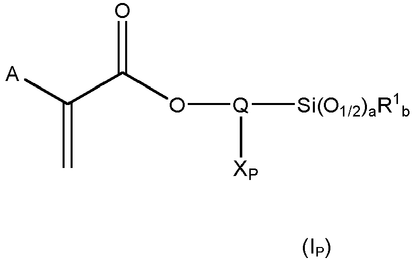
wobei für die Struktureinheiten A, Q, X_{P} und R¹ in jeder der strukturell identischen ersten Siloxaneinheiten unabhängig voneinander gilt:
A bedeutet H oder CH₃,
Q bedeutet eine den Substituenten X_{P} tragende Verknüpfungsgruppe,
R¹ bedeutet einen Alkylrest mit insgesamt 1 bis 4 Kohlenstoffatomen
und
X_{P} eine reaktive Gruppe
wobei
b 0, 1 oder 2 bedeutet und
a 3-b bedeutet,
- Umsetzen der intermediären Polysiloxanverbindung in ein oder mehr Schritten unter Reaktion der reaktiven Gruppe X_{P}, so dass die Polysiloxanverbindung nach einem der Ansprüche 1 bis 4 gebildet wird.

13. Verfahren zur Herstellung eines härtbaren Dentalmaterials nach einem der Ansprüche 5 oder 6 mit den Schritten:
- Bereitstellen oder Herstellen einer oder mehrerer Polysiloxanverbindungen nach einem der Ansprüche 1 bis 4,
- Bereitstellen oder Herstellen einer, mehrerer oder sämtlicher Substanzen wie in Anspruch 6 definiert,
- Herstellen eines einkomponentigen oder mehrkomponentigen Systems umfassend die bereitgestellten bzw. hergestellten Polysiloxanverbindung(en) sowie die bereitgestellten bzw. hergestellten Substanz(en) sowie optional zusätzliche Substanzen,
wobei vorzugsweise in mehrkomponentigen Systemen die eine Polymerisation auslösenden Substanzen so auf separate Komponenten verteilt sind, dass eine Polymerisation der Polysiloxanverbindungen durch Vermischen der besagten Komponenten ausgelöst wird.

14. Verfahren zur Herstellung eines gehärteten Dentalmaterials nach Anspruch 7, mit folgenden Schritten:
- Bereitstellen oder Herstellen eines härtbaren Dentalmaterials nach einem der Ansprüche 5 bis 6,
- Polymerisation der im Dentalmaterial enthaltenen Polysiloxanverbindung sowie gegebenenfalls weiterer im Dentalmaterial enthaltener polymerisierbarer Bestandteile.

## Claims

1. Polysiloxane compound comprising
(A) one, two, three or more than three structurally in each case identical first siloxane units selected from the group consisting of siloxane units of the general formula (I) 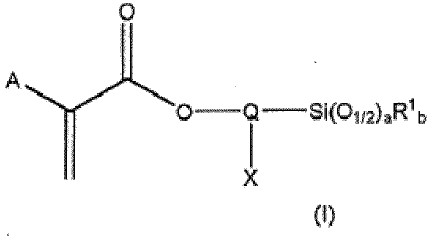 wherein for the structural units A, Q, X and R¹ in each of the structurally identical first siloxane units independently of each other:
A denotes H or CH₃,
Q denotes a linking group carrying the substituent X,
R¹ denotes an alkyl radical having a total of 1 to 4 carbon atoms
and
X is selected from the group consisting of
branched, saturated, unsubstituted alkyl radical having a total of 7 to 18 carbon atoms,
unbranched, saturated, unsubstituted alkyl radical having a total of 7 to 18 carbon atoms,
unsubstituted or alkyl-substituted aryl radical having a total of 10 to 18 carbon atoms, wherein the alkyl substituent in the alkyl-substituted aryl radical is
branched and saturated
or
unbranched and saturated
and
Z-(CO)-R², wherein for the structural units Z and R² herein independently of each other and independently of the meaning of the structural units A, Q and R¹;
Z denotes O, S or NH, preferably O and
R² is selected from the group consisting of
branched, saturated, unsubstituted alkyl radical having a total of 6 to 18 carbon atoms,
unbranched, saturated, unsubstituted alkyl radical having a total of 6 to 18 carbon atoms,
unsubstituted or alkyl-substituted aryl radical having a total of 9 to 18 carbon atoms, wherein the alkyl substituent in the alkyl-substituted aryl radical is
branched and saturated or
unbranched and saturated,
wherein
b denotes 0, 1 or 2 and
a denotes 3-b
and
wherein a carbon atom of an alkyl radical can be linked to another carbon atom of this alkyl radical to form a ring.

2. Polysiloxane compound, preferably according to claim 1, comprising
(A) one, two, three or more than three structurally in each case identical first siloxane units selected from the group consisting of siloxane units of the general formula (Ia) 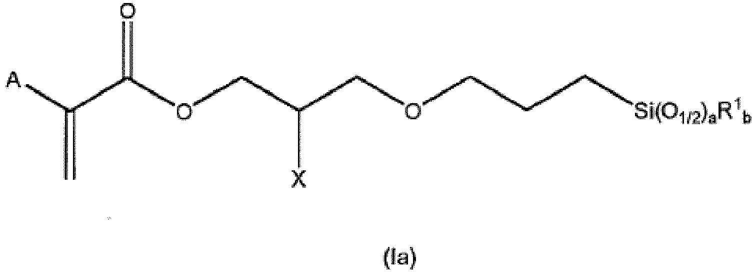 wherein for the structural units A, X and R¹ in each of the structurally identical first siloxane units independently of each other:
A denotes H or CH₃,
R¹ denotes an alkyl radical having a total of 1 to 4 carbon atoms
and
X is selected from the group consisting of
branched, saturated, unsubstituted alkyl radical having a total of 7 to 18 carbon atoms, preferably 11 to 18 carbon atoms, particularly preferably 11 to 14 carbon atoms,
unbranched, saturated, unsubstituted alkyl radical having a total of 7 to 18 carbon atoms, preferably 11 to 18 carbon atoms, particularly preferably 11 to 14 carbon atoms,
unsubstituted or alkyl-substituted aryl radical having a total of 10 to 18 carbon atoms, preferably 11 to 18 carbon atoms, particularly preferably 11 to 14 carbon atoms, wherein the alkyl substituent in the alkyl-substituted aryl radical is
branched and saturated
or
unbranched and saturated
and
Z-(CO)-R², wherein for the structural units Z and R² herein independently of each other and independently of the meaning of the structural units A and R¹:
Z denotes O, S or NH, preferably O
and
R² is selected from the group consisting of
branched, saturated, unsubstituted alkyl radical having a total of 6 to 18 carbon atoms, preferably 10 to 18 carbon atoms, particularly preferably 10 to 14 carbon atoms,
unbranched, saturated, unsubstituted alkyl radical having a total of 6 to 18 carbon atoms, preferably 10 to 18 carbon atoms, particularly preferably 10 to 14 carbon atoms,
unsubstituted or alkyl-substituted aryl radical having a total of 9 to 18 carbon atoms, preferably 10 to 18 carbon atoms, particularly preferably 10 to 14 carbon atoms, wherein the alkyl substituent in the alkyl-substituted aryl radical is
branched and saturated or
unbranched and saturated,
wherein
b denotes 0, 1 or 2 and
a denotes 3-b
and
(B) one, two, three or more than three second, structurally in each case identical siloxane units which differ structurally from the first siloxane unit.

3. Polysiloxane compound according to one of the preceding claims, comprising
(A) one, two, three or more than three structurally in each case identical first siloxane units selected from the group consisting of siloxane units of the general formula (Ic) 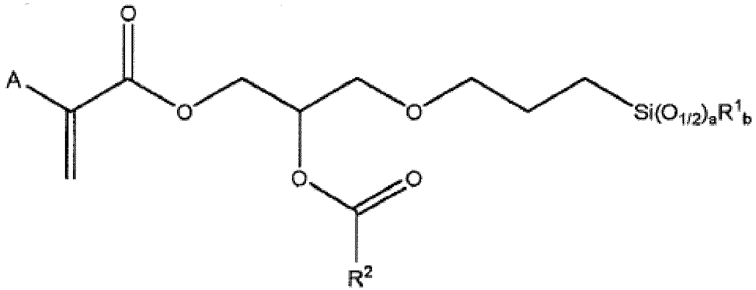 wherein for the structural units A, R¹ and R² in each of the structurally identical first siloxane units independently of each other:
A denotes H or CH₃,
R¹ denotes an alkyl radical having a total of 1 to 4 carbon atoms,
and
R² is selected from the group consisting of
branched, saturated, unsubstituted alkyl radical having a total of 6 to 18 carbon atoms, preferably 10 to 18 carbon atoms, particularly preferably 10 to 14 carbon atoms,
unbranched, saturated, unsubstituted alkyl radical having a total of 6 to 18 carbon atoms, preferably 10 to 18 carbon atoms, particularly preferably 10 to 14 carbon atoms,
unsubstituted or alkyl-substituted aryl radical having a total of 9 to 18 carbon atoms, preferably 10 to 18 carbon atoms, particularly preferably 10 to 14 carbon atoms, wherein the alkyl substituent in the alkyl-substituted aryl radical is
branched and saturated
or
unbranched and saturated,
wherein
b denotes 0, 1 or 2 and
a denotes 3-b
and
(B) one, two, three or more than three second, structurally in each case identical siloxane units which differ structurally from the first siloxane unit.

4. Polysiloxane compound according to one of the preceding claims, wherein R² is selected from the group consisting of
1-naphthyl,
4-tert-butyl-1-phenyl
and
n-undecanyl.

5. Curable dental material comprising
- one or more than one polysiloxane compound according to one of the preceding claims
and
- one or more than one compound which is not a polysiloxane compound according to one of the preceding claims.

6. Curable dental material according to claim 5, further comprising one, two, more than two or all the substances from the group consisting of:
- dental organic filler particles which are preferably radiopaque and/or nanoscale in size,
- dental inorganic filler particles which are preferably radiopaque and/or nanoscale in size,
- dental organically surface-modified inorganic filler particles which are preferably radiopaque and/or nanoscale in size,
- rheology auxiliaries,
- polymerisation initiators, preferably photoinitiators,
- chemical compounds as catalysts or constituents of catalyst systems,
- colouring agents, preferably coloured pigments,
- stabilisers, in particular daylight stabilisers,
- inhibitors,
- activators,
- molecular weight regulators,
- preservatives,
- surface-active substances,
- microbicides, preferably bactericides,
- organic monomers which can preferably be polymerised by free radicals and which are not polysiloxanes according to the invention, preferably for reaction with the polysiloxane compound according to the invention,
- organic polymers and oligomers and compounds having high molecular weights, preferably plasticisers,
- thickening agents and
- dental medicaments.

7. Cured dental material obtainable from a curable dental material according to one of claims 5 to 6 by means of polymerisation of the polysiloxane compound contained in the dental material and optionally further polymerisable constituents contained in the dental material.

8. Use of a cured dental material according to claim 7 for producing dental components or precursors of dental components, preferably for producing dental components or precursors of dental components by means of computer-aided design (CAD) and/or computer-aided manufacturing (CAM).

9. Polysiloxane compound according to one of claims 1 to 4 or curable dental material according to one of claims 5 or 6 or cured dental material according to claim 7, for use in a therapeutic method, wherein a polysiloxane compound according to one of claims 1 to 4 is preferably employed as a polymerisable constituent of a curable dental material.

10. Polysiloxane compound according to one of claims 1 to 4 or curable dental material according to one of claims 5 to 6 or cured dental material according to claim 7, for use according to claim 9, for specific use
in a therapeutic method for temporary or permanent filling of a dental cavity
or
in a therapeutic method as a
dental filling material,
dental lining material,
dental adhesive (bonding),
as a flowable composite material (flow material),
as a fissure sealant,
as a crown material,
as an inlay and/or onlay,
as a bridge material
and/or as a core build-up material.

11. Kit comprising
- one, two or more than two curable dental materials according to one of claims 5 to 6
and/or
- one, two or more than two base pastes and one, two or more than two catalyst pastes, wherein the one base paste or the two or more than two base pastes in each case and independently of each other comprises or comprise one or more than one polysiloxane compound according to one of claims 1 to 4.

12. Method for producing a polysiloxane compound according to one of claims 1 to 4, having the following steps:
- producing or providing an intermediate polysiloxane compound comprising
- (A_{P}) one, two, three or more than three structurally in each case identical first intermediate siloxane units selected from the group consisting of siloxane units of the general formula (I_{P}) 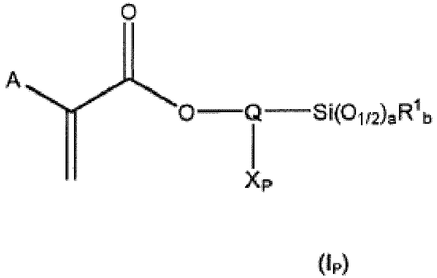 wherein for the structural units A, Q, X_{P} and R¹ in each of the structurally identical first siloxane units independently of each other:
A denotes H or CH₃,
Q denotes a linking group carrying the substituent X_{P}
R¹ denotes an alkyl radical having a total of 1 to 4 carbon atoms
and
X_{P} denotes a reactive group
wherein
b denotes 0, 1 or 2 and
a denotes 3-b,
- reacting the intermediate polysiloxane compound in one or more steps with reaction of the reactive group X_{P}, so that the polysiloxane compound according to one of claims 1 to 4 is formed.

13. Method for producing a curable dental material according to one of claims 5 or 6, having the steps:
- providing or producing one or more polysiloxane compounds according to one of claims 1 to 4,
- providing or producing one, more or all the substances as defined in claim 6,
- producing a one-component or multicomponent system comprising the polysiloxane compound(s) provided or produced and the substance(s) provided or produced and optionally additional substances,
wherein preferably in multicomponent systems the substances which trigger a polymerisation are distributed among separate components such that a polymerisation of the polysiloxane compounds is triggered by mixing said components.

14. Method for producing a cured dental material according to claim 7, having the following steps:
- providing or producing a curable dental material according to one of claims 5 to 6,
- polymerising the polysiloxane compound contained in the dental material and optionally further polymerisable constituents contained in the dental material.

## Revendications

1. Composé de polysiloxane comprenant
(A) un, deux, trois ou plus que trois premiers motifs de siloxane respectivement identiques sur le plan structurel choisis parmi le groupe constitué de motifs de siloxane de formule générale (I) 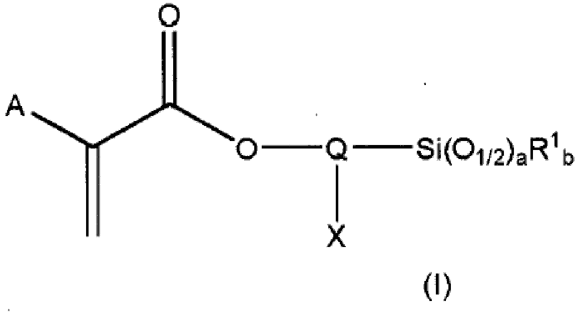 dans lequel, pour les motifs de siloxane A, Q, X et R¹ dans chacun des premiers motifs de siloxane identiques sur le plan structurel, s'applique indépendamment les uns des autres ce qui suit :
A signifie H ou CH₃,
Q signifie un groupe de liaison portant les substituants X,
R¹ signifie un radical alkyle comprenant au total 1 à 4 atomes de carbone
et
X est choisi parmi le groupe constitué
- d'un radical alkyle ramifié, saturé, non substitué comprenant au total 7 à 18 atomes de carbone,
- d'un radical alkyle non ramifié, saturé, non substitué comprenant au total 7 à 18 atomes de carbone,
- d'un radical aryle non substitué ou substitué par un radical alkyle comprenant au total 10 à 18 atomes de carbone, dans lequel le substituant alkyle dans le radical aryle substitué par un radical alkyle est
- ramifié et saturé
ou
- non ramifié et saturé,
et
Z-(CO)-R², dans lequel s'applique ici ce qui suit pour les motifs structurels Z et R² indépendamment l'un de l'autre et indépendamment de la signification des motifs structurels A, Q, et R¹ :
Z signifie 0, S, ou NH, de préférence 0
et
R² est choisi parmi le groupe constitué de
- d'un radical alkyle ramifié, saturé, non substitué comprenant au total 6 à 18 atomes de carbone,
- d'un radical alkyle non ramifié, saturé, non substitué comprenant au total 6 à 18 atomes de carbone,
- d' un radical aryle non substitué ou substitué par un radical alkyle comprenant au total 9 à 18 atomes de carbone, dans lequel le substituant alkyle dans le radical aryle substitué par un radical alkyle est
- ramifié et saturé
ou
- non ramifié et saturé,
dans lequel
b signifie 0, 1 ou 2 et
que a signifie 3-b
et
dans lequel un atome de carbone d'un radical alkyle peut être lié à un autre atome de carbone dudit radical alkyle en formant un cycle.

2. Composé de polysiloxane, de préférence selon la revendication 1, comprenant
(A) un, deux, trois ou plus de trois premiers motifs de siloxane respectivement identiques sur le plan structurel choisis parmi le groupe constitué de motifs de siloxane de formule générale (Ia) 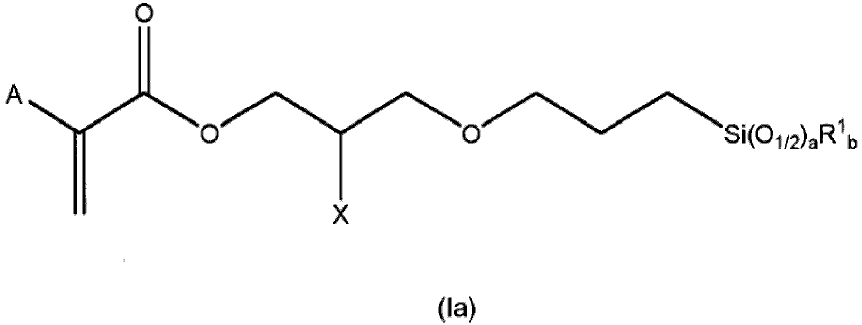 dans lequel, pour les motifs structurels A, X et R¹ dans chacun des premiers motifs de siloxane identiques sur le plan structurel, s'applique ce qui suit indépendamment les uns des autres :
A signifie H ou CH₃,
R¹ signifie un radical alkyle comprenant au total 1 à 4 atomes de carbone,
et
X est choisi parmi le groupe constitué
- d'un radical alkyle ramifié, saturé, non substitué comprenant au total 7 à 18 atomes de carbone, de préférence 11 à 18 atomes de carbone, de manière particulièrement préférée 11 à 14 atomes de carbone,
- d'un radical alkyle non ramifié, saturé, non substitué comprenant au total 7 à 18 atomes de carbone, de préférence 11 à 18 atomes de carbone, de manière particulièrement préférée 11 à 14 atomes de carbone,
- d' un radical aryle non substitué ou substitué par un radical alkyle comprenant au total 10 à 18 atomes de carbone, de préférence 11 à 18 atomes de carbone, de manière particulièrement préférée 11 à 14 atomes de carbone, dans lequel le substituant alkyle dans le radical aryle substitué par un radical alkyle est
- ramifié et saturé
ou
- non ramifié et saturé
et
Z-(CO)-R², dans lequel ici s'applique ce qui suit pour les motifs structurels Z et R² indépendamment l'un de l'autre et indépendamment de la signification des motifs structurels A et R¹ :
Z signifie 0, S ou NH, de préférence 0,
et
R² est choisi parmi le groupe constitué
- d'un radical alkyle ramifié, saturé, non substitué comprenant au total 6 à 18 atomes de carbone, de préférence 10 à 18 atomes de carbone, de manière particulièrement préférée 10 à 14 atomes de carbone,
- d'un radical alkyle non ramifié, saturé, non substitué comprenant au total 6 à 18 atomes de carbone, de préférence 10 à 18 atomes de carbone, de manière particulièrement préférée 10 à 14 atomes de carbone,
- d'un radical aryle non substitué ou substitué par un radical alkyle comprenant au total 9 à 18 atomes de carbone, de préférence 10 à 18 atomes de carbone, de manière particulièrement préférée 10 à 14 atomes de carbone, dans lequel le substituant alkyle dans le radical aryle substitué par un radical alkyle est
- ramifié et saturé
ou
- non ramifié et saturé,
dans lequel
b signifie 0, 1 ou 2 et
a signifie 3-b
et
(B) un, deux, trois ou plus de trois deuxièmes motifs de siloxane respectivement identiques sur le plan structurel, qui sont différents sur le plan structurel du premier motif de siloxane.

3. Composé de polysiloxane selon l'une quelconque des revendications précédentes, comprenant
(A) un, deux, trois ou plus de trois premiers motifs de siloxane respectivement identiques sur le plan structurel choisis parmi le groupe constitué de motifs de siloxane de formule générale (Ic) 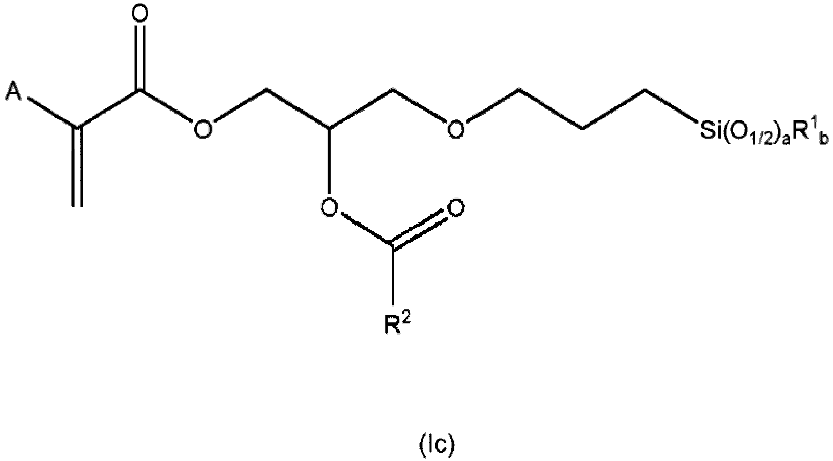 dans lequel, pour les motifs structurels A, R¹ et R² dans chacun des premiers motifs de siloxane identiques sur le plan structurel, s'applique ce qui suit indépendamment les uns des autres :
A signifie H ou CH₃,
R¹ signifie un radical alkyle comprenant au total 1 à 4 atomes de carbone,
et
R² est choisi parmi le groupe constitué
- d'un radical alkyle ramifié, saturé, non substitué comprenant au total 6 à 18 atomes de carbone, de préférence 10 à 18 atomes de carbone, de manière particulièrement préférée 10 à 14 atomes de carbone,
- d'un radical alkyle non ramifié, saturé, non substitué comprenant au total 6 à 18 atomes de carbone, de préférence 10 à 18 atomes de carbone, de manière particulièrement préférée 10 à 14 atomes de carbone,
- d'un radical aryle non substitué ou substitué par un radical alkyle comprenant au total 9 à 18 atomes de carbone, de préférence 10 à 18 atomes de carbone, de manière particulièrement préférée 10 à 14 atomes de carbone, dans lequel le substituant alkyle dans le radical aryle substitué par un radical alkyle est
- ramifié et saturé
ou
- non ramifié et saturé,
dans lequel
b signifie 0, 1 ou 2 et
a signifie 3-b
et
(B) un, deux, trois ou plus de trois deuxièmes motifs de siloxane respectivement identiques sur le plan structurel, qui sont différents sur le plan structurel du premier motif de siloxane.

4. Composé de polysiloxane selon l'une quelconque des revendications précédentes, dans lequel R² est choisi parmi le groupe constitué de
1-naphthyle,
4-tert.-butyl-1-phényle,
et
n-undécanyle.

5. Matériau à usage dentaire durcissable comprenant
- un ou plus d' un composé de polysiloxane selon l'une quelconque des revendications précédentes
et
- un ou plus d'un composé, qui n'est pas un composé de polysiloxane selon l'une quelconque des revendications précédentes.

6. Matériau à usage dentaire durcissable selon la revendication 5, comprenant en outre une, deux, plus de deux ou toutes les substances issues du groupe constitué :
- de particules de charge organiques à usage dentaire, qui sont de préférence opaques aux rayons X et/ou nanométriques,
- de particules de charge inorganiques à usage dentaire, qui sont de préférence opaques aux rayons X et/ou nanométriques,
- de particules de charges inorganiques modifiées en surface de manière organique à usage dentaire, qui sont de préférence opaques aux rayons X et/ou nanométriques,
- d'adjuvants rhéologiques,
- d'initiateurs de polymérisation, de préférence de photoinitiateurs,
- de composés chimiques en tant que catalyseurs ou constituants de systèmes catalyseurs,
- de colorants, de préférence de pigments colorés,
- de stabilisateurs, en particulier de stabilisateurs à la lumière du jour,
- d'inhibiteurs,
- d'activateurs,
- de régulateurs de poids moléculaire,
- d'agents de conservation,
- de substances tensioactives,
- de microbicides, de préférence de bactéricides,
- de monomères organiques, de préférence polymérisables de manière radicalaire, qui ne sont pas des polysiloxanes selon l'invention, de préférence servant à la mise en réaction avec le composé de polysiloxane selon l'invention,
- de polymères organiques et d'oligomères et de composés présentant des poids moléculaires élevés, de préférence de plastifiants,
- d'agents épaississants, et
- de médicaments à usage dentaire.

7. Matériau à usage dentaire durci pouvant être obtenu à partir d'un matériau dentaire durcissable selon l'une quelconque des revendications 5 à 6 au moyen d'une polymérisation du composé de polysiloxane contenu dans le matériau à usage dentaire ainsi qu'éventuellement d'autres constituants polymérisables contenus dans le matériau à usage dentaire.

8. Utilisation d'un matériau dentaire durci selon la revendication 7 servant à fabriquer des composants à usage dentaire ou des précurseurs de composants à usage dentaire, de préférence servant à fabriquer des composants à usage dentaire ou des précurseurs de composants à usage dentaire au moyen d'une mise en forme assistée par ordinateur (CAD, computer-aided design) et/ou d'une production assistée par ordinateur (computer-aided manufacturing, CAM).

9. Composé de polysiloxane selon l'une quelconque des revendications 1 à 4 ou matériau à usage dentaire durcissable selon l'une quelconque des revendications 5 à 6 ou matériau à usage dentaire durci selon la revendication 7, destiné à être utilisé lors d'un procédé thérapeutique, dans lequel un composé de polysiloxane selon l'une quelconque des revendications 1 à 4 est employé de préférence en tant que constituant polymérisable d'un matériau à usage dentaire durcissable.

10. Composé de polysiloxane selon l'une quelconque des revendications 1 à 4 ou matériau à usage dentaire durcissable selon l'une quelconque des revendications 5 à 6 ou matériau à usage dentaire durci selon la revendication 7 destiné à être utilisé selon la revendication 9, aux fins de l'utilisation spécifique dans un procédé thérapeutique servant à remplir de matière temporaire ou permanente une cavité dentaire
ou dans un procédé thérapeutique en tant
- que matériau de remplissage à usage dentaire,
- que matériau de remplissage partiel à usage dentaire,
- qu'adhésif à usage dentaire (bonding),
- que matériau composite pouvant s'écouler (flow-material),
- que matériau d'obturation de fissures,
- que matériau de couronne,
- qu'inlay et/ou onlay,
- que matériau de bridge,
- et/ou que matériau de reconstitution de moignons.

11. Ensemble comprenant
- un, deux ou plus de deux matériaux à usage dentaire durcissables selon l'une quelconque des revendications 5 à 6, et/ou
- une, deux ou plus de deux pâtes de base et une, deux ou plus de deux pâtes de catalyseur, dans lequel une pâte de base ou les deux pâtes de base ou plus comprennent respectivement et indépendamment les unes des autres un ou plus d'un composé de polysiloxane selon l'une quelconque des revendications 1 à 4.

12. Procédé servant à fabriquer un composé de polysiloxane selon l'une quelconque des revendications 1 à 4, comprenant les étapes suivantes consistant à :
- fabriquer ou fournir un composé de polysiloxane intermédiaire comprenant
- (A_{P}) un, deux, trois ou plus de trois premiers motifs de siloxane intermédiaires respectivement identiques sur le plan structurel choisis parmi le groupe constitué de motifs de siloxane de formule générale (I_{P}) 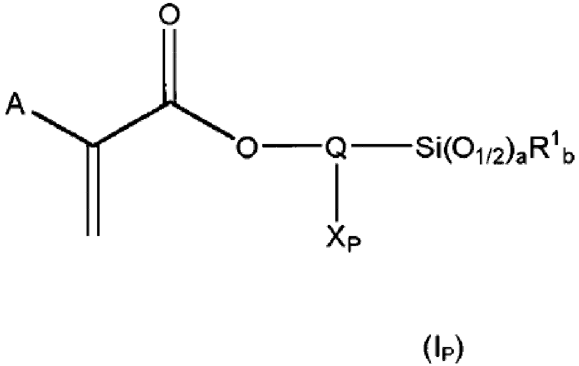 dans lequel, pour les motifs structurels A, Q, Xₚ et R¹ dans chacun des premiers motifs de siloxane identiques sur le plan structurel, s'applique ce qui suit indépendamment les uns des autres :
A signifie H ou CH₃,
Q signifie un groupe de liaison supportant le substituant Xₚ,
R¹ signifie un radical alkyle comprenant au total 1 à 4 atomes de carbone,
et
Xₚ un groupe réactif,
dans lequel
b signifie 0, 1 ou 2, et
a signifie 3-b,
- faire réagir le composé de polysiloxane intermédiaire lors d'une ou de plusieurs étapes en faisant réagir le groupe réactif Xₚ de sorte que le composé de polysiloxane selon l'une quelconque des revendications 1 à 4 est formé.

13. Procédé servant à fabriquer un matériau à usage dentaire durcissable selon l'une quelconque des revendications 5 ou 6 comprenant les étapes consistant à :
- fournir ou fabriquer un ou plusieurs composés de polysiloxane selon l'une quelconque des revendications 1 à 4 ;
- fournir ou fabriquer une, plusieurs ou toutes les substances telles que définies dans la revendication 6 ;
- fabriquer un système à un composant ou à plusieurs composants comprenant le/les composé(s) de polysiloxane fourni(s) ou fabriqué(s) ainsi que la ou les substance(s) fournie(s) ou fabriquée(s) ainsi qu'en option des substances supplémentaires
dans lequel de préférence les substances déclenchant une polymérisation sont réparties de préférence dans des systèmes à plusieurs composants de telle manière sur des composants séparés qu'une polymérisation des composés de polysiloxane est déclenchée par le mélange desdits composants.

14. Procédé servant à fabriquer un matériau à usage dentaire durci selon la revendication 7, comprenant des étapes qui suivent consistant à :
- fournir ou fabriquer un matériau à usage dentaire durcissable selon l'une quelconque des revendications 5 à 6 ;
- effectuer la polymérisation du composé de polysiloxane contenu dans le matériau à usage dentaire ainsi éventuellement que d'autres constituants polymérisables contenus dans le matériau à usage dentaire.
